Europäisches Patentamt
European Patent Office
Office européen des brevets

(11) Publication number: 0 174 956 B1

# EUROPEAN PATENT SPECIFICATION

(45) Date of publication of patent specification: 16.08.90

(51) Int. Cl.[5]: C 07 C 215/20, C 07 C 217/54, A 61 K 31/21

(21) Application number: 85901281.7

(22) Date of filing: 28.02.85

(86) International application number: PCT/US85/00322

(87) International publication number: WO 85/04168 26.09.85 Gazette 85/21

## (54) SOFT -g(b)-ADRENERGIC BLOCKING AGENTS.

(30) Priority: 14.03.84 US 589359

(43) Date of publication of application: 26.03.86 Bulletin 86/13

(45) Publication of the grant of the patent: 16.08.90 Bulletin 90/33

(84) Designated Contracting States: AT BE CH DE FR GB LI LU NL SE

(56) References cited: none

(73) Proprietor: BODOR, Nicholas S.
7211 Southwest 97th Lane
Gainsville, FL 32608 (US)

(72) Inventor: BODOR, Nicholas S.
7211 Southwest 97th Lane
Gainsville, FL 32608 (US)

(74) Representative: Pendlebury, Anthony et al
PAGE, WHITE & FARRER 54 Doughty Street
London WC1N 2LS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Soft β-adrenergic Blocking Agents

Field of the Invention

The present invention provides novel compounds containing at least one aromatic nucleus which are soft β-adrenergic blocking agents, useful in the treatment or prevention of cardiovascular disorders and in the treatment of glaucoma.

Background of the Invention

Many β-adrenergic blocking agents are known and used; unfortunately, however, these agents are generally subject to facile oxidative metabolic degradations. Many of the metabolites also possess significant β-blocking activity and, due to their different pharmacokinetic properties, make dosing and optimization of therapy difficult. The metabolites of bufuralol, for example, have longer biological half-lives than the parent drug. Francis et al, *Biomed. Mass. Spectrometry, 3,* 281—285 (1976). Consequently it is difficult to determine an adequate dose of the known β-blockers for administration, especially when administering them therapeutically to patients suffering from angina pectoris, hypertension or unexpected arrhythmias during surgical operations. It would be most desirable to design β-blockers which would be metabolized in a simple, predictable and controllable manner in one step to an inactive metabolite, regardless of the conditions of the patient and other drugs used. This would necessitate, however, avoiding oxidative metabolism.

The present inventor has previously devised a general soft drug approach having such objectives, one specific aspect of that approach being the inactive metabolite method. Bodor, Proceedings of the 2nd IUPAC-IUPHAR Symposium on Strategy in Drug Research, Noordwijkerhout, J. A. Keverling Buisman (ed.), Elsevier Scientific Publishing Company, Amsterdam, 1982; Bodor, Belgian Patent No. 889,563, 03 November 1981; *Chem. Abstr.* 97:6651n (1982). The principles of the inactive metabolite approach are:

(1) Start the design process with a known inactive metabolite of a drug.

(2) Alter the metabolite to obtain a structure that resembles (isosteric and/or isoelectronic) the starting or an analogous drug.

(3) Design the structure and metabolism of the new soft compound in such a way as to yield the starting inactive metabolite in one step without going through toxic intermediates.

(4) Control transport and binding properties as well as the rate of metabolism and phamacokinetics by molecular manipulations in the activation stage. However, it is not necessary to wait for an inactive metabolite to be isolated; it may be possible to design the inactive metabolite during the general drug design process based on knowledge of structural requirements for activity as well as elimination and enzymatic cleavage.

Recently, American Hospital Supply Corporation's International Application No. PCT/US81/01514, published under International Publication No. W082/01869 on 10 June 1982, described a series of short acting β-adrenergic blocking compounds containing ester moieties which are structurally related to some of the compounds of the present invention. The PCT application does not, however, appear to address the problem addressed by the present invention, i.e. how to design β-blockers which would be metabolized in one simple, predictable, controllable step to an inactive metabolite while avoiding oxidative metabolism; moreover, the esters of the PCT application are generally less complex than those described herein below. See also Erhardt et al, *J. Med. Chem., 25,* 1408—1412 (1982). Similar simple alkyl esters were described much earlier, e.g. Barrett et al United States Patent No. 3,663,607, issued 16 May 1972. See also Japanese Patent Application No. 230664/1982, filed 27 December 1982.

Summary and Objects of the Invention:

In view with the foregoing, it is an object of this invention to provide a new class of β-adrenergic agents which will be metabolized in a simple, predictable and controllable manner in one step to an inactive metabolite. It is another object of the present invention to provide new β-adrenergic agents which are not subject to significant oxidative metabolism. It is another object of this invention to apply the present inventor's general inactive metabolite approach to the design of novel soft β-blockers.

These and other objects of the present invention are achieved by providing compounds of the formula

$$RO\overset{O}{\overset{\|}{C}}—C_nH_{2n}—Ar—OCH_2\overset{OH}{\overset{|}{C}}HCH_2NHR_1 \qquad (I)$$

or a pharmaceutically acceptable acid addition salt thereof, wherein:

n is one;

R is $C_6$—$C_{18}$ polycycloalkyl-$C_pH_{2p}$- wherein p is 0, 1, 2, or 3; $C_6$—$C_{18}$ polycycloalkenyl-$C_pH_{2p}$- wherein p is defined as above; —$CH_2$—X—$R_2$ wherein X is S, SO or $SO_2$ and $R_2$ is $C_1$—$C_7$ alkyl or $C_3$—$C_{12}$ cycloalkyl;

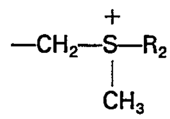

wherein $R_2$ is defined as above;

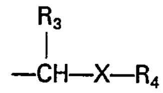

wherein X is defined as above, and wherein $R_3$ is $C_1$—$C_7$ alkyl and $R_4$ is $C_1$—$C_7$ alkyl or wherein $R_3$ and $R_4$ taken together represent $—(CH_2)_m—$ wherein m is 3 or 4 and $—(CH_2)_m—$ is optionally substituted by one to three $C_1$—$C_7$ alkyl;

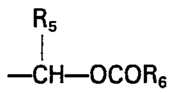

wherein $R_5$ is hydrogen or $C_1$—$C_7$ alkyl and $R_6$ is unsubstituted or substituted $C_1$—$C_7$ alkyl, $C_3$—$C_{12}$ cycloalkyl, $C_3$—$C_{12}$ cycloalkenyl or $C_2$—$C_8$ alkenyl, the substituents being selected from the group consisting of halo, $C_1$—$C_7$ alkoxy, $C_1$—$C_7$ alkylthio, $C_1$—$C_7$ alkylsulfinyl, $C_1$—$C_7$ alkylsulfonyl,

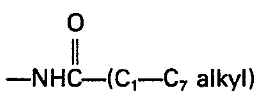 and 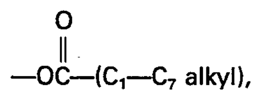,

or $R_6$ is substituted or unsubstituted phenyl or benzyl, the substituents being selected from the group consisting of $C_1$—$C_7$ alkyl, $C_1$—$C_7$ alkoxy, halo, carbamoyl, $C_2$—$C_8$ alkoxycarbonyl, $C_2$—$C_8$ alkanoyloxy, $C_1$—$C_7$ haloalkyl, mono($C_1$—$C_7$ alkyl)amino, di($C_1$—$C_7$ alkyl)amino, mono($C_1$—$C_7$ alkyl)carbamoyl, di($C_1$—$C_7$ alkyl)carbamoyl, $C_1$—$C_7$ alkylthio, $C_1$—$C_7$ alkylsulfinyl and $C_1$—$C_7$ alkylsulfonyl;

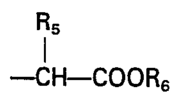

wherein $R_5$ and $R_6$ are defined as above; or

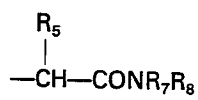

wherein $R_5$ is defined as above and $R_7$ and $R_8$, which can be the same or different, are each hydrogen, $C_1$—$C_7$ alkyl, $C_3$—$C_{12}$ cycloalkyl, phenyl or benzyl, or $R_7$ and $R_8$ are combined such that $—NR_7R_8$ represents the residue of a saturated monocyclic secondary amine;

$R_1$ is $C_1$—$C_7$ alkyl; and

Ar is a phenylene radical which is unsubstituted or which is substituted by $C_1$—$C_7$ alkyl, $C_1$—$C_7$ alkyl-O—$C_1$—$C_7$ alkylene-, $C_2$—$C_8$ alkenyl, $C_1$—$C_7$ alkyl-S—, $C_2$—$C_8$ alkenyl-O—, $C_3$—$C_{12}$ cycloalkyl, $C_1$—$C_7$ alkyl-CONH—, $C_1$—$C_7$ alkyl-CO— or $H_2NCO$-$C_1$—$C_7$ alkylene-; or

Ar is a radical of the formula

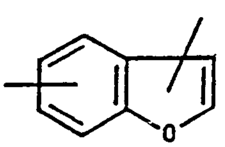, 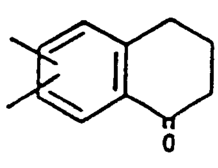, 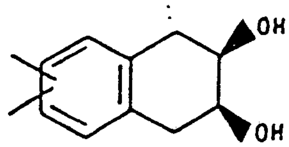, 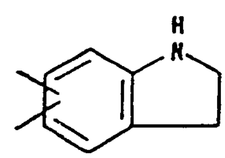,

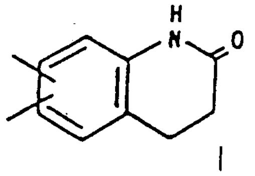, 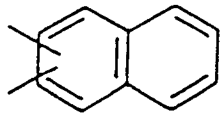 or 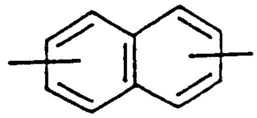.

or Ar is a phenylene-$C_1$—$C_3$ alkylene-phenyl radical.

The compounds of the present invention avoid the disadvantages of many known β-blockers resulting from their active metabolites. The compounds of formula (I) are rationally designed so that when administered to a mammal they are subjected to an enzymatic hydrolytic (i.e. esterase) process which is

much faster than oxidative metabolism and which leads to hydrolyzed metabolites which possess very little or no β-adrenergic activity. Thus, for example, in the case of a group of preferred metoprolol derivatives of the invention, such are designed to hydrolytically deactivate to the phenylacetic acid derivative, i.e. the compound of the formula

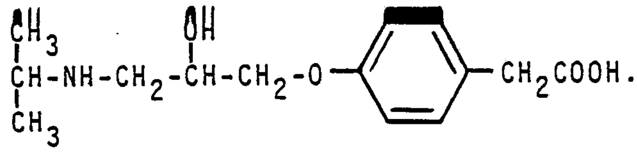

which is an inactive metabolite of metoprolol and the major metabolite found in the urine. [See, for example, Borg et al, *Acta Pharmacol. Toxicol., 36* (Suppl. V), 125—135 (1975).] The rate of hydrolysis deactivation may be controlled by the structure of the esters.

Accordingly, the compounds of the present invention exhibit excellent β-blocking efficacy without adverse side effects for a certain duration, dependent only on the hydrolytic metabolism rate. As a consequence, it is easy to control and maintain adequate therapeutic action with a desirable onset time using the present compounds. The onset time is usually short compared with conventional β-adrenergic blocking agents; therefore, the instant compounds are expected to be of particular use in the treatment of arrhythmias, ischemic heart disease and hypertension, possibly attending surgical operations. The instant compounds are also expected to be of special value when used in the treatment of glaucoma, since they will not only reduce intraocular pressure when applied locally to the eye, but because of their "soft" nature will deactivate during absorption and thus avoid the typical β-blocker systemic side effects which attend use of known β-blockers for this purpose.

Detailed Description of the Invention

With respect to the various groups encompassed by the generic terms used in this specification, the following definitions and explanations are applicable:

Illustrative divalent radicals represented by Ar include the following:

(1) phenylene, i.e. a radical of the formula

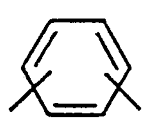 ,

especially 1,4-phenylene and 1,3-phenylene, optionally bearing one or more substituents such as $C_1$—$C_7$ alkyl, e.g. $CH_3CH_2$— or $CH_3$—; $C_1$—$C_7$ alkyl-O—$C_1$—$C_7$ alkylene-, e.g. $CH_3OCH_2CH_2$—; $C_2$—$C_8$ alkenyl, e.g. $CH_2$=CH—$CH_2$-; $C_1$—$C_7$alkyl-S—, e.g. $CH_3$—S; $C_2$—$C_8$ alkenyl-O—, e.g. $CH_2CH$=$CH_2$-O—; $C_3$—$C_{12}$ cycloalkyl, e.g. cyclopentyl; $C_1$—$C_7$ alkyl-CONH—, e.g. $CH_3CH_2CH_2CONH$— or $CH_3CONH$—; $C_1$—$C_7$ alkyl-CO—, e.g. $CH_3CO$—; and/or $H_2NCO$—$C_1$—$C_7$ alkylene-; e.g. $H_2NCO$—$CH_2$—;

(2) divalent fused ring systems such as:

(a) 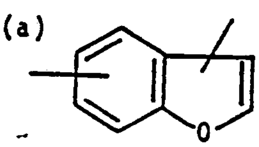 , especially 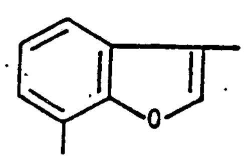 ;

(b) 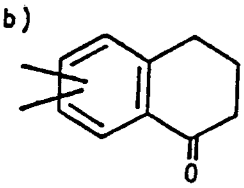 , especially 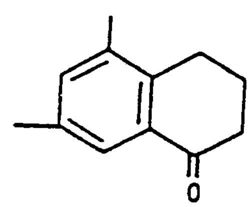 ;

(c) 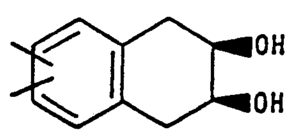 , especially 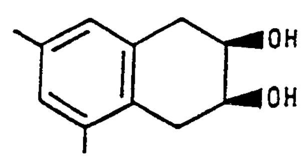 ;

(d) 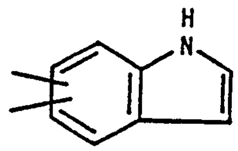 , especially 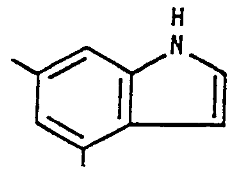 ;

(e) 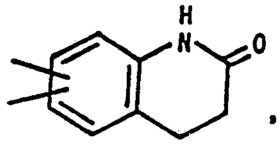 , especially 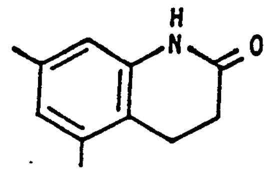 ;

and (f) 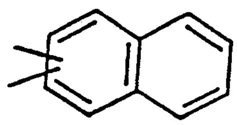 or

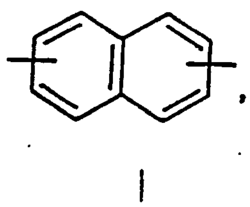 , especially 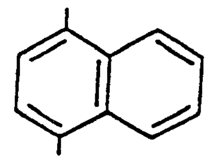 ;

and

(3) phenylene-$C_1$—$C_3$ alkylene-phenyl radicals such as

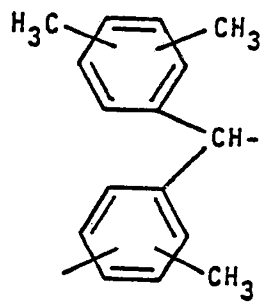

Preferably, the divalent radical represented by AR is selected such that the

$$RO\overset{O}{\overset{\|}{C}}—C_nH_{2n}-$$

grouping in formula (I) either simply replaces a hydrogen atom in the corresponding ring of a known β-blocker or replaces a non-critical ring substituent in the corresponding known β-blocker, e.g. a lower alkyl, lower alkyl-O-lower alkylene-, lower alkyl-CONH- or $H_2NCO$-lower alkylene- substituent. Particularly preferred compounds of the invention bear such a structural relationship to metoprolol, bufuralol, alprenolol, bunolol, xipranolol, nadolol, tiprenolol, oxprenolol, penbutolol, pindolol, carteolol, propranolol, acebutolol, atenolol and practolol.

The alkyl, alkenyl and alkylene groupings encompassed by any of the structural variables in formula (I) can be straight or branched-chain groups containing the indicated number of carbon atoms. The term "lower' used in conjunction with such radicals indicates that they may contain up to 7 carbon atoms.

Specific examples of alkyl radicals encompassed by formula (I), whether as specific values for $R_1$ or as a portion of an R or Ar group, include methyl, ethyl, propyl, butyl, pentyl, hexyl and heptyl and their branched-chain isomers, e.g. isopropyl, isobutyl and tert-butyl. Preferred values for $R_1$ are isopropyl and tert-butyl.

The alkenyl radicals encompassed by various R and Ar values can be exemplified by vinyl, propenyl and butenyl and the branched-chain groups having 3 or more carbon atoms.

The alkylene moieties encompassed by various values for Ar are typified by methylene, ethylene, trimethylene, 2-methyltrimethylene, 2,2-dimethyltrimethylene, 1-methyltrimethylene, methylmethylene, ethylmethylene, tetramethylene, pentamethylene, hexamethylene and the like.

The alkoxy, alkylthio, alkylsulfinyl, alkylsulfonyl, alkoxycarbonyl, alkanoyloxy, monoalkylamino, dialkylamino, monoalkylcarbamoyl and dialkylcarbamoyl groupings are of the type

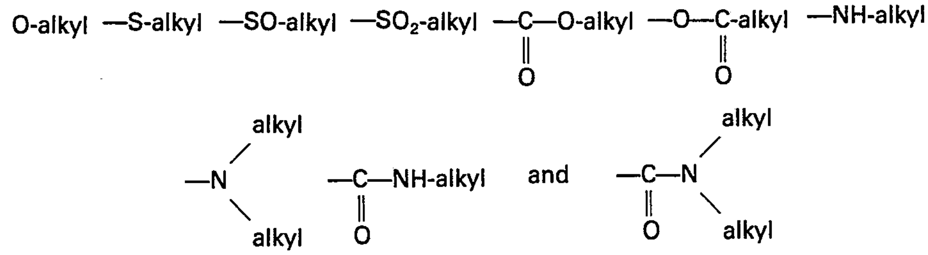

respectively, wherein alkyl is as hereinbefore defined and exemplified.

The halo substituents can be chloro, bromo, iodo or fluoro. The haloalkyl substituents can be monohaloalkyl or polyhaloalkyl, straight or branched-chain, substituted with from 1 to 3 halogen atoms, the term "halogen" as used herein including chlorine, bromine, iodine or fluorine. Specific examples of the contemplated monohaloalkyl and polyhaloalkyl groups include chloromethyl, dichloromethyl, trichloromethyl, bromomethyl, fluoromethyl, difluoromethyl, trifluoromethyl, 1-fluoroethyl, 1-chloroethyl, 2-chloroethyl, 2,2,2-trichloroethyl, 2,2,2-trifluoroethyl, 1,2-dichloroethyl, 1-chloropropyl, 3-chloropropyl, 1-chlorobutyl, 1-chloropentyl, 1-chlorohexyl, 4-chlorobutyl and the like.

When a cycloalkyl radical is present in formula (I) as a portion of Ar or as a portion of R (e.g. when R is $-CH_2-X-R_2$ wherein $R_2$ is $C_3-C_{12}$ cycloalkyl), then such a cycloalkyl radical can contain 3 to 8 ring atoms and may optionally bear one or more, preferably one to four, alkyl substituents. Examples of such cycloalkyl groups include cyclopropyl, 2-methylcyclopropyl, 3-ethylcyclopropyl, 2-butylcyclopropyl, 3-pentylcyclopropyl, 2-hexylcyclopropyl, cyclobutyl, 2-methylcyclobutyl, 2,3-dimethylcyclobutyl, 3-butylcyclobutyl, 4-hexylcyclobutyl, 2,3,3-trimethylcyclobutyl, 3,3,4,4-tetramethylcyclobutyl, cyclopentyl, 2-methylcyclopentyl, 3-ethylcyclopentyl, 4-butylcycloethylenepentyl, 5-methylcyclopentyl, 3-pentylcyclopentyl, 4-hexylcyclopentyl, 2,3-dimethylcyclopentyl, 2,2,5,5-tetramethylcyclopentyl, 2,3,4-trimethylcyclopentyl, 2,4-dimethyl-3-ethylcyclopentyl, 2,2,3,4,4-pentamethylcyclopentyl, 2,3-dimethyl-3-propylcyclopentyl, cyclohexyl, 2,6-dimethylcyclohexyl, 3,3,5,5-tetramethylcyclohexyl, 2-methylcyclohexyl, 2-ethylcyclohexyl, 4-propylcyclohexyl, 5-butylcyclohexyl, 2,3-dimethylcyclohexyl, 2,4-dimethylcyclohexyl, 2,5-dimethylcyclohexyl, 2,3,4-trimethylcyclohexyl, 2,3-dimethyl-5-ethylcyclohexyl, 2,5-dimethyl-6-propylcyclohexyl, 2,4-dimethyl-3-butylcyclohexyl, 2,2,4,4-tetramethylcyclohexyl, 3,3,6,6-tetramethylcyclohexyl, 3,3,4,5,5-pentamethylcyclohexyl, 3,3,4,5,5,6-hexamethylcyclohexyl, 3,3,5-trimethyl-4-ethylcyclohexyl, 3,4,4-trimethyl-5-propylcyclohexyl, cycloheptyl, 3-methylcycloheptyl, 5-propylcycloheptyl, 6-butylcycloheptyl, 7-methylcycloheptyl, cyclooctyl, 2-methylcyclooctyl, 3-ethylcyclooctyl, 3,3,4-trimethylcyclooctyl, 3,3,5,5-tetramethylcyclooctyl and the like. When a cycloalkenyl radical is present in formula (I) as a portion of R, corresponding unsaturated radicals such as cyclopentenyl and cyclohexenyl and the like are contemplated.

The polycycloalkyl-$C_pH_{2p}$- radicals represented by R are bridged saturated alicyclic hydrocarbon systems consisting of two or more rings, optionally bearing one or more alkyl substituents and having a total of 6 to 18 carbon atoms in the ring portion. The corresponding bridged unsaturated alicyclic hydrocarbon systems are intended by the term "$C_6-C_{18}$ polycycloalkenyl-$C_pH_{2p}$-". Such polycycloalkyl and polycycloalkenyl radicals are exemplified by adamantyl(especially 1- or 2-adamantyl), adamantylmethyl(especially 1-adamantylmethyl), adamantylethyl(especially 1- adamantylethyl), bornyl, norbornyl (e.g. *exo*-norbornyl or *endo*-norbornyl), norbornenyl (e.g. 5-norbornen-2-yl), norbornylmethyl (e.g. 2-norbornylmethyl) and norbornylethyl (e.g. 2-norbornylethyl). Thus, in the case of polycyclic radicals, p is preferably 0, 1 or 2.

When R in formula (I) is

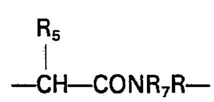

wherein $-NR_7R_8$ represents the residue of a saturated monocyclic secondary amine, such monocycles preferably have 5 to 7 ring atoms optionally containing another hetero atom (—O—, —S— or —N—) in addition to the indicated nitrogen atom, and optionally bear one or more substituents such as phenyl, benzyl and methyl. Illustrative of residues of saturated monocyclic secondary amines whch are encompassed by the $-NR_7R_8$ term are morpholino, 1-pyrrolidinyl, 4-benzyl-1-piperazinyl, perhydro-1,2,4-oxathiazin-4-yl, 1- or 4-piperazinyl, 4-methyl-1-piperazinyl, piperidino, hexamethyleneimino, 4-phenylpiperidino, 2-methyl-1-pyrazolidinyl, 1- or 2-pyrazolidinyl, 3-methyl-1-imidazolidinyl, 1- or 3-imidazolidinyl, 4-benzylpiperidino and 4-phenyl-1-piperazinyl.

The compounds of formula (I) can be prepared by reacting a starting material of the formula

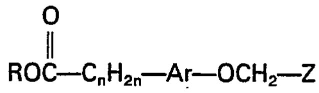

(II)

wherein R, n and Ar are defined as above and Z is

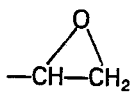 or 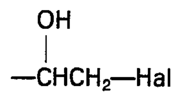

wherein Hal is a halogen atom, especially Cl or Br, with a primary amine of the formula

$$NH_2R_1 \qquad (III)$$

wherein $R_1$ is defined as above. This process is preferably carried out in the presence of an inert solvent, although it can be carried out in the absence of solvent. While time and temperature are not critical factors, the reaction is generally conducted at a temperature from about room temperature to about 200°C (preferably from about 60 to 120°C), for a period of time from about 30 minutes to 24 hours. Suitable inert solvents include ethers such as dioxane, tetrahydrofuran and ethylene glycol monomethyl ether; aromatic hydrocarbons such as benzene, toluene or xylene; lower alcohols such as methanol, ethanol and isopropanol; and esters such as ethyl acetate, dimethylformamide and dimethylsulfoxide. The process is frequently carried out in the presence of a basic compound, for example, an inorganic base such as sodium carbonate, sodium hydroxide, sodium bicarbonate, sodium amide or sodium hydride; or an oranic base such as triethylamine, tripropylamine, pyridine, quinoline, DBN (1,5-diazabiscyclo[4.3.0]non-5-ene), DBU(1,8-diazabicyclo[5.4.0]undec-7-ene) or Dabco (1,4-diazabicyclo[(2.2.2]octane or triethylenediamine). The ratio of the amounts of reactants used can be selected over a wide range. It is, however, generally desirable for the amine of formula (III) to be used in an equimolar quantity or in an excess amount, preferably from an equimolar quantity to about seven times the molar quantity.

The starting materials of formula (II) can be conveniently prepared by reacting the corresponding compounds of the formula

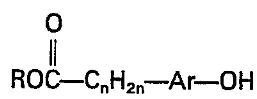

(IV)

wherein R, n and Ar are defined as before, with an epihalogenohydrin of the formula

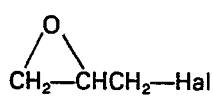

(V)

wherein Hal is a halogen atom, especially Cl or Br. This process can be carried out in the presence of a suitable basic compound, for example an inorganic base such as sodium hydroxide, potassium hydroxide, sodium carbonate, potassium carbonate, sodium methoxide, sodium ethoxide, sodium hydride, sodium metal, potassium metal or sodium amide, or an organic basic compund such as piperidine, pyridine, triethylamine, DBN, DBU or Dabco, in the presence or absence of a solvent. Suitable solvents are, for example, a lower alcohol such as methanol, ethanol or isopropanol; a ketone such as acetone; an ether such as dioxane, tetrahydrofuran or ethylene glycol monomethyl ether; or an aromatic hydrocarbon such as benzene, toluene or xylene. In this reaction, the epihalogenohydrin of formula (V) ordinarily can be used in an equimolar to excess quantity, preferably about 5 to 15 times the molar quantity of the formula (IV) starting material. The process can be conducted at a temperature between about 0 and 150°C, preferably at from about 50 to about 100°C. Usually, the reaction product is a mixture of the corresponding (2,3-epoxy)propoxy and 3-halogeno-2-hydroxypropoxy compounds encompassed by formula (II) and can be used as such to prepare the formula (I) compounds.

The starting materials of formula (IV) can be prepared by esterification of carboxylic acids of the formula

$$HOOC—C_nH_{2n}—Ar—OH \qquad (VI)$$

wherein n and Ar are defined as before, or the corresponding acid chlorides or acid anhydrides, by reaction with an alcohol of the formula

$$ROH \qquad (VII)$$

wherein R is defined as before. Reaction conditions, solvents and the like vary with the particular reactants

employed. Generally speaking, when an acid of formula (VI) is utilized as the starting material, the process is conducted in the presence of a conventional esterification catalyst, e.g. an inorganic acid such as hydrogen chloride gas, concentrated sulfuric acid, phosphoric acid, polyphosphoric acid, boron trifluoride or hypochlorous acid, or an organic acid such as trifluoroacetic acid, trifluoromethanesulfonic acid, naphthalenesulfonic acid, p-toluenesulfonic acid, benzenesulfonic acid, ethanesulfonic acid, trifluoromethanesulfonic acid anhydride, thionyl chloride, acetone dimethyl acetal and the like. A cation exchange resin may also be used as the catalyst. The reaction can be carried out in the presence or absence of solvent, at a temperature between about −20 to about 200°C, preferably between about 0 and about 150°C, for a period of time from about 10 minutes to about 20 hours. If a solvent is employed, an inert solvent is desirable, for example, an aromatic hydrocarbon such as benzene, toluene or xylene; a halogenated hydrocarbon such as chloroform, dichloromethane, dichloroethane or carbon tetrachloride; or an ether such as diethyl ether, tetrahydrofuran, dioxane or ethylene glycol monomethyl ether. The ratio of amount of a compound of formula (VI) to an alcohol of the formula ROH is not subject to any specific retriction and may be suitably selected from a wide range. While usually it is desirable that the latter is used in an excess quantity in the absence of the solvent, in the presence of the solvent it is desirable that the latter is used in an equimolar to 5 times the molar quantity of the former, more preferably an equimolar to 2 times the molar quantity of the former. This reaction can advantageously be effected by using a drying agent such as anhydrous calcium chloride, anhydrous cupric sulfate, anhydrous calcium sulfate, phosphorus pentoxide or the like.

Alternatively, an acid of formula (VI) can be converted to the correspnding salt of the type

$$M^{+-}OOC—C_nH_{2n}—Ar—OH \quad (VIII)$$

where n and Ar are defined as before and $M^+$ is $Na^+$ or the like; that salt can then be reacted with a halide of the formula

$$R—Hal \quad (IX)$$

where R is defined as before and Hal is halogen, preferably Cl or Br, to afford the corresponding starting material of formula (IV).

The compounds of the invention which contain a sulfinyl (SO) or sulfonyl ($SO_2$) group can be prepared by oxidation of the corresponding sulfur-containing compounds of the invention. Thus, for example, a compound of formula (I) wherein R is $—CH_2—X—R_2$ wherein X is S can be treated with one equivalent of *m*-chloroperbenzoic acid to afford the corresponding sulfinyl derivative, while treatment of the thio compound with two equivalents of *m*-chloroperbenzoic acid yields the corresponding sulfonyl derivative. Thus, the compounds of formula (I) wherein R contains a sulfur atom, e.g. the compounds in which R is $—CH_2—X—R_2$ or

$$\overset{\displaystyle R_3}{\underset{|}{}}\\ —CH—X—R_4$$

wherein X is S and $R_2$, $R_3$ and $R_4$ are defined above, are of value not only as soft β-adrenergic agents but also as chemical intermediates to the corresponding soft drugs in which X is SO or $SO_2$.

The compounds of the present invention can also be prepared by esterification of the corresponding acid of the formula

$$HOOC—C_nH_{2n}—Ar—OCH_2\overset{OH}{\overset{|}{C}}HCH_2NHR_1 \quad (X)$$

wherein n, Ar and $R_1$ are defined as above, under conditions similar to the esterification of a compound of formula (VI) as described above.

Equivalent to the compounds of formula (I) for the purposes of this invention are the corresponding acid addition salts formed with pharmaceutically acceptable acids. Illustrative of such acids are inorganic acids such as hydrochloric acid, sulfuric acid, hydrobromic acid and the like; and organic acids such as acetic acid, oxalic acid, succinic acid, maleic acid, fumaric acid, malic acid, tartaric acid, citric acid, malonic acid, methanesulfonic acid, benzoic acid and the like.

The compounds prepared by the procedures detailed above can easily be isolated and purified by usual separation means, e.g. solvent extraction, dilution, recrystallization, column chromatography or preparative thin-layer chromatography.

The compounds of the present invention also include their optical isomers.

For therapeutic use, e.g. in the treatment of angina pectoris and arrhythmias, a compound of formula (I) or its salt can be conveniently administered in the form of a pharmaceutical composition containing the formula (I) compound or its salt and a pharmaceutically acceptable carrier therefor. Suitable carriers vary

with the desired form of the pharmaceutical composition and may include diluents or excipients such as fillers, binders, wetting agents, disintegrators, surface-active agents, lubricants and the like.

The compound of the invention or its salt may be formulated together with the carrier into any desired unit dosage form. Typical unit dosage forms include tablets, pills, powders, solutions, suspensions, emulsions, granules, capsules, suppositories; injectable solutions and suspensions are particularly preferred.

In the preparation of tablets, carriers which are widely used in this field can be employed, e.g. excipients such as lactose, sucrose, sodium chloride, glucose solution, urea, starch, calcium carbonate, kaolin, crystalline cellulose and silicic acid; binding agents such as water, ethanol, propanol, simple syrup, glucose, starch solution, gelatin solution, carboxymethyl cellulose, shellac, methyl cellulose, calcium phosphate and polyvinylpyrrolidone; disintegrators such as dried starch, sodium alginate, agar-agar powder, laminalia powder, sodium bicarbonate, calcium carbonate, Tweens, sodium lauryl sulfate, stearic acid monoglyceride, starch and lactose; disintegration inhibitors such as sucrose, stearin, coconut butter and hydrogenated oil; absorption accelerators such as quaternary ammonium bases and sodium lauryl sulfate; wetting agents such as glycerin and starch; adsorbing agents such as starch, lactose, kaolin, bentonite and colloidal silicic acid; and lubricants such as purified talc, stearic acid salts, boric acid powder, Macrogol and solid polyethylene glycol.

In the preparation of pills, carriers which are known and widely used in this field can also be used, for example, excipients such as glucose, lactose, starch, coconut butter, hydrogenated vegetable oils, kaolin and talc; binders such as powdered gum arabic, powdered tragacanth, gelatin and ethanol; and disintegrators such as laminaria and agar-agar. In the case of tablets, they can be further coated with the usual coating materials to make sugar-coated tablets, gelatin film-coated tablets, tablets coated with enteric coatings, tablets coated with films or double-layered tablets and multi-layered tablets.

In order to form suppositories, carriers which are known and widely used in this field can also be used, for example, polyethylene glycols, coconut butter, higher alcohols, esters of higher alcohols, gelatin and semi-synthesized glycerides.

In order to prepare formulations suitable for injection, solutions and suspensions are sterilized and are preferably isotonic to blood. In making injectable preparations, carriers which are commonly used in this field can also be used, for example, water, ethyl alcohol, propylene glycol, ethoxylated isostearyl alcohol, polyoxylated isostearyl alcohol, polyoxyethylene sorbitol and sorbitane esters. In these instances, adequate amounts of sodium chloride, glucose or glycerin can be added to make the preparations isotonic.

Furthermore, the usual dissolving agents, buffers, analgesic agents and preservatives can be added, as well as coloring materials, perfumes, seasoning agents, sweetening agents and other medicines, to the pharmaceutical compositions, if necessary or if desired.

The amount of a compound of formula (I) or its acid addition salt to be present in the pharmaceutical composition, e.g. for use in treatment of angina pectoris and arrhythmias, can suitably be selected from a wide range, but usually 1 to 70% by weight of the total composition.

As to the route of administration, e.g. for angina pectoris, same will vary with the particular composition used. For example, tablets, pills, solutions, suspensions, emulsions, granules and capsules can be administered orally; and injectable preparations can be administered intravenously, either alone or mixed wih injection transfusions such as glucose solutions and amino acid solutions. If necessary, the injectable preparations can be administered separately, by the intramuscular, intracutaneous, subcutaneous or intraperitoneal route.

The dosage of the compounds of the present invention is selected according to the usage, purpose and conditions of symptoms. For example, when these compounds are administered therapeutically to patients suffering from angina pectoris, hypertension, or unexpected arrhythmias during surgical operation, usually 0.5—6.0 mg/kg of the compound of general formula (I) or its acid addition salt may be administered.

The present compounds also may be continuously administered at suitable intervals, including 30 minutes to 60 minutes.

The compounds of the present invention and their salts reduce intraocular pressure when applied topically/locally to the eye, thus are of particular use in the treatment of patients with glaucoma or in the treatment of other patients who require lowering of ocular pressure (such as patients with elevated intraocular pressure who may be at risk of developing glaucoma). The instant compounds and their salts can be conveniently administered for these purposes by formulating the selected β-blocker, in an effective intraocular pressure lowering amount, together with a non-toxic ophthalmically acceptable carrier therefor. Suitable carriers will be apparent to those skilled in the art of ophthalmic formulations. Obviously, the choice of suitable carriers will depend on the exact nature of the particular dosage form desired, e.g. whether the β-blocker is to be formulated into an ophthalmic solution or suspension (typically for use as eye drops), an ophthalmic ointment or cream or an ophthalmic gel. Preferred dosage forms are solutions, which contain a major amount of water in addition to the active ingredient. Minor amounts of other ingredients such as pH adjusters (e.g. a base such as NaOH), emulsifiers or dispersing agents, buffering agents, preservatives, wetting agents and jelling agents (e.g. methylcellulose) may also be present. Most preferably, the ophthalmic composition is a sterile, isotonic, buffered aqueous solution. Generally speaking, the ophthalmic composition containing the instant β-blockers may be prepared and may contain the various inert ingredients or carriers as previously described in the patent or non-patent literature as

being suitable for ophthalmic compositions comprising known β-blockers such as timolol and labetolol. The amount of the β-blocker of this invention which will be present in the ophthalmic composition will of course vary with the particular β-blocker employed and the type of formulation selected. Generally speaking, the composition will contain 0.01 to 5% of a compound of formula (I), preferably 0.25 to 2.5%; in other words, each ml of solution will contain 0.1 to 50 mg, preferably 2.5 to 25 mg, of the free base. The dose administered ophthalmically will be selected according to the particular compound employed and the size and condition of the patient, but in any event will be a quantity sufficient to cause a significant reduction in intraocular pressure.

In order to further illustrate the present invention and the advantages thereof, specific examples of compounds of formula (I) are given below, it being understood that these examples are intended only as illustrative and are not in any way limitative. Also exemplified below are the preparation of selected starting materials. In all the examples to follow, all melting points are uncorrected and were obtained by using electrothermal capillary melting point apparatus.

Example 1

To a solution of 4-hydroxyphenylacetic acid (7.6 g, 0.05 mol) in ethanol (100 mL) was added potassium hydroxide (2.8 g, 0.05 mol), with stirring and ice cooling. The white crystalline potassium salt which was separated by filtration melted at about 275—277°C and was obtained in 90.5% yield.

The potassim salt of 4-hydroxyphenylacetic acid (9.5 g, 0.05 mL) was suspended in dimethylformamide (25 mL). Chloromethyl pivalate (7.53 g, 0.05 mol) was added and the reaction mixture was stirred at room temperature for 24 hours. The reaction mixture was poured into ice water and extracted with ethyl acetate (100 mL). The ethyl acetate layer was washed, first with aqueous 5% $NaHCO_3$ (100 mL) and then twice with water, then was dried over anhydrous $MgSO_4$, filtered and evaporated to yield pivalyloxymethyl 4-hydroxyphenylacetate, melting at 159—161°C (yield 70.2%).

Example 2

Pivalyloxymethyl 4-hydroxyphenylacetate (7.0 g, 0.05 mol) was dissolved in acetone (50 mL) and epichlorohydrin (40 mL) was added. That mixture was refluxed in the presence of DBU (1 mL, 0.007 mol) for 2 hours, then was allowed to stir at room temperature overnight. The reaction mixture was evaporated *in vacuo*. The epoxide residue was dissolved in acetonitrile (80 mL), isopropylamine (20 mL, 0.23 mol) was added and the reaction mixture was stirred overnight at room temperature, refluxed for 4 hours and evaporated. The free amine base was purified by extraction with acid and base from ether. After evaporation of the ether, the residual oil was partitioned between ethyl ether and 1M aqueous KOH solution, the ether layer was washed with 1M HCl, the acidic layer was basified with 1M aqueous KOH solution, and fresh anhydrous ethyl ether was added. The ether layer was separated, dried over anhydrous $MgSO_4$ and filtered. An equivalent amount of oxalic acid in acetone was added dropwise. The oxalate salt which separated was filtered and dried. It melted at 123—124°C. The free base, pivalyloxymethyl 4-(2-hydroxy-3-isopropylamino)propoxyphenylacetate, has the structure:

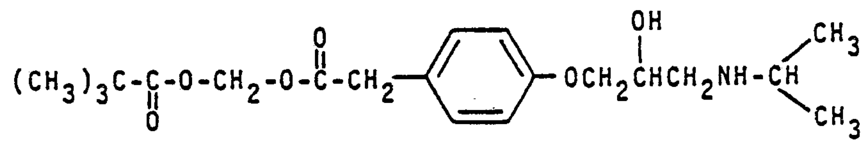

The identity of the product was confirmed by NMR and elemental analysis.

Example 3

1-Adamantaneethanol (10 g, 0.05 mol), 4-hydroxyphenylacetic acid (8.43 g, 0.05 mol) and p-toluenesulfonic acid (1 g, 0.006 mol) were refluxed in benzene (100 mL) with continuous water separation for 8 hours, then allowed to stir overnight. The residue was washed, first with 5% aqueous sodium bicarbonate solution (50 mL) and then twice with water (50 mL), then was dried over anhydrous $MgSO_4$ and filtered. the filtrate was concentrated *in vacuo* to give (adamant-1-yl)ethyl 4-hydroxyphenylacetate.

(Adamant-1-yl)ethyl 4-hydroxyphenylacetate was taken up in epichlorohydrin (50 mL) and refluxed for 2 hours in the presence of DBU (1 mL, 0.007 mol). The residue was dissolved in acetonitrile (100 mL) and refluxed with isopropylamine (20 mL, 0.23 mol) for 4 hours. The reaction mixture was evaporated *in vacuo* and the residue was partitioned between ethyl ether (100 mL) and 0.5 N aqueous $NaHCO_3$ solution (100 mL). The ether layer was separated and 1N HCl (30 mL) was added to it. The aqueous layer was separated, 1M aqueous KOH solution (30 mL) was added and the solution was extracted with ether (100 mL). The aqueous layer was discarded and the ether layer was dried over anhydrous $MgSO_4$, filtered and evaporated *in vacuo*. The oily product was dissolved in acetone (25 mL) and an equivalent quantity of oxalic acid in acetone (25 mL) was added dropwise, with stirring. The acetone was evaporated *in vacuo* and the residue was partitioned between ether (100 mL) and 1M aqueous KOH solution (100 mL). The ether layer was separated, dried over anhydrous $MgSO_4$, filtered and concentrated *in vacuo*. The oily residue was dissolved in acetone (25 mL) and an equivalent amount of oxalic acid in acetone (25 mL) was added dropwise, with

stirring. The product which separated was removed by filtration, dried and recrystallized from acetone. The oxalate salt of (adamant-1-yl)ethyl 4-(2-hydroxy-3-isoproylamino)propoxyphenylacetate thus obtained melted at 95—97°C. Yield 70.9%. The free base has the structure:

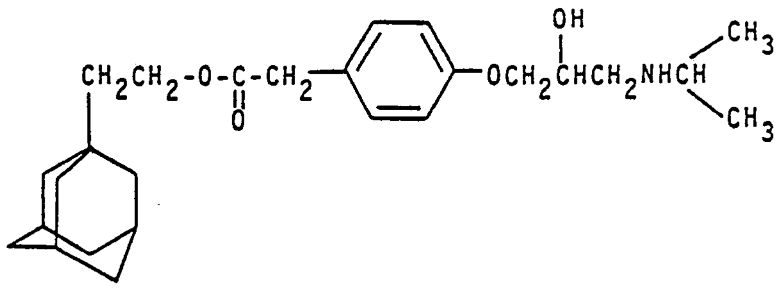

NMR and elemental analysis were consistent with the assigned structure.

Example 4

1-Adamantanemethanol (8.31 g, 0.05 mol), 4-hydroxyphenylacetic acid (7.6 g, 0.05 mol) and p-toluenesulfonic acid (1 g, 0.006 mol) were refluxed in benzene (100 mL) with continuous water separation for 8 hours. The reaction mixture was allowed to cool with stirring overnight, then was washed, first with water (100 mL), then with aqueous 5% $NaHCO_3$ solution (100 mL), then twice more with water (100 mL). Drying over anhydrous $MgSO_4$, filtering and concentrating *in vacuo* afforded (adamant-1-yl)methyl 4-hydroxyphenylacetate, which was then combined with epichlorohydrin (50 mL) and refluxed for 2 hours in the presence of DBU (1 mL, 0.007 mol). Excess epichlorohydrin was removed in vacuo. The oily residue was dissolved in acetonitrile (100 mL) and combined with isopropylamine (20 mL, 0.23 mol). The resultant reaction mixture was refluxed for 4 hours. The oily brown residue was partitioned between ethyl ether (100 mL) and 1M aqueous KOH solution (100 mL). The ether layer was separated and to it was added 1N HCl (30 mL). The resultant acid layer was separated and to it were added ethyl ether (100 mL) and 1M aqueous KOH solution (30 mL). The resultant ether layer was separated, dried over anhydrous $MgSO_4$, filtered and evaporated *in vacuo* to give the free amine base as a crude product. The crude product (5.16 g, 72.5% yield) was dissolved in acetone (25 mL), and oxalic acid (1.8 g) in acetone (25 mL) was added dropwise, with stirring. The mixture was allowed to stir for 24 hours with additional acetone. The acetone solution was evaporated and the oily residue was partitioned between ether (100 mL) and 1M aqueous KOH solution (100 mL). The ether solution was dried over anhydrous $MgSO_4$, filtered and concentrated. The oily residue was dissolved in acetone and an equivalent amount of oxalic acid in acetone was added dropwise. The oxalate salt of (adamant-1-yl)methyl 4-(2-hydroxy-3-isopropylamino)propoxyphenylacetate which separated was removed and dried. It melted at 55—57°C. NMR and elemental analysis were consistent with structure. The free base has the formula:

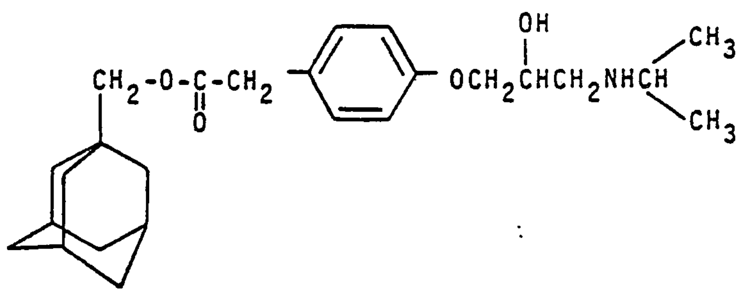

Example 5

2-Norboranemethanol (6.34 g, 0.05 mol), 4-hydroxyphenylacetic acid (7.6 g, 0.05 mol), p-toluenesulfonic acid (2 g, 0.006 mol) and benzene (100 mL) were refluxed with continuous water separation for 8 hours. The solvent was evaporated and the residue was dissolved in ethyl acetate (100 mL) and washed, first with 5% aqueous $NaHCO_3$ solution (100 mL), then twice with water (200 mL). Drying over anhydrous $MgSO_4$, filtering and evaporation left an oily residue, identified by NMR as the desired (norborn-2-yl)methyl 4-hydroxyphenylacetate. That product was taken up in acetone (50 mL), epichlorohydrin (40 mL) was added, and the reaction mixture was refluxed for 2 hours in the presence of DBU (2 mL, 0.007 mol). The reaction mixture was evaporated *in vacuo* and the residual oil was taken up in acetonitrile (100 mL). Isopropylamine (20 mL, 0.23 mol) was added and the reaction mixture was refluxed for 4 hours, then evaporated to give the free amine base as an oil. The oil was dissolved in acetone (50 mL) and an equivalent amount of oxalic acid in acetone (25 mL) was added dropwise with stirring. A white crystalline by-product melting at 198—200°C separated and was discarded. The acetone was evaporated *in vacuo* and the oil thus

obtained was partitioned between ethyl ether (100 mL) and 1M aqueous KOH solution (100 mL). The ether layer was separated and 1M HCl (50 mL) was added. The acidic layer was separated, neutralized with 1M aqueous KOH solution (50 mL) and extracted with ethyl ether (100 mL). The ether layer was dried over anhydrous $MgSO_4$, filtered and evaporated to give the free amine base as an oil. Water (25 mL) was added to the oil; then oxalic acid (5.4 g, 0.01 mol) in acetone (25 mL) was added dropwise, with constant stirring. The oxalate salt of (norborn-2-yl)methyl 4-(2-hydroxy-3-isopropylamino)-propoxyphenylacetate which separated after cooling was washed with acetone (200 mL) and crystallized from a mixture of ethyl acetate and acetone. It melted at 68—70°C. The corresponding free base has the structure:

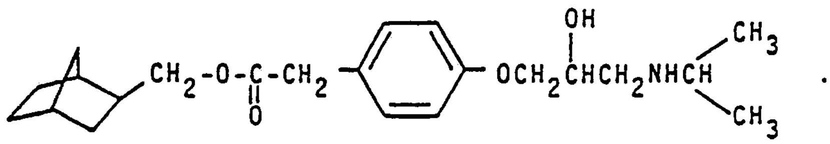

Elemental analysis and NMR were consistent with the assigned structure.

Example 6

The potassium salt of 4-hydroxyphenylacetic acid (9.5 g, 0.1 mol) was taken up in a mixture of dimethylsulfoxide (80 mL) and hexamethylphosphoramide (5.37 g, 0.1 mol). 2-Choroacetamide (13.5 g, 0.3 mol) was added and the reaction mixture was first stirred at room temperature for 24 hours, then heated for 1 hour. The reaction mixture was evaporated, then cooled. The product which separated was taken up in ethylacetate (150 mL), stirred, filtered and evaporated. The oily residue was partitioned between ethyl acetate (100 mL) and water (100 mL). The organic layer was separated, dried over anhydrous $MgSO_4$, filtered and evaporated to give the desired ester, carbamoylmethyl 4-hydroxyphenylacetate, melting at 85—87°C, in 48.8% yield.

Carbamoylmethyl 4-hydroxyphenylacetate (10.4 g, 0.01 mol) was taken up in acetone. Epichlorohydrin (40 mL) was added and the reaction mixture was refluxed for 2 hours in the presence of DBU (2 mL, 0.014 mol). The mixture was then evaporated to give an oil, which was dissolved in acetonitrile (100 mL) and then refluxed with isopropylamine (20 mL, 0.23 mol) for 4 hours. The resultant mixture was evaporated *in vacuo* to give the desired free amine base as an oil. That oil was dissolved in acetone and an equivalent amount of oxalic acid in acetone was added. The acetone solution was concentrated and then partitioned between ethyl ether (100 mL) and 1M aqueous KOH solution (50 mL). The ether layer was separated and extracted with 1M HCl (50 mL). The resultant acidic solution was neutralized with 1M aqueous KOH solution and extracted with ethyl ether. The ether layer was separated, dried over anhydrous $MgSO_4$, filtered and evaporated. The residue was dissolved in acetone and an equivalent amount of oxalic acid in acetone was added. The oxalate salt of carbamoylmethyl 4-(2-hydroxy-3-isopropylamino)-propoxyphenylacetate which separated after cooling was removed by filtration and dried. It melted at 93—95°C. The corresponding free base has the structural formula:

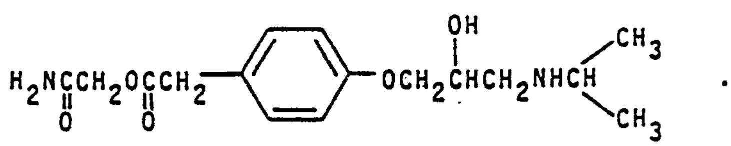

NMR and elemental analysis were consistent with the assigned structure.

Example 7

Use of the starting materials indicated below in the general esterification procedure described in Example 3, first paragraph, affords the indicated products:

| ROH | + | HOOC-$C_nH_{2n}$-Ar-OH | → | ROOC-$C_nH_{2n}$-Ar-OH | |
|---|---|---|---|---|---|
| $CH_3SCH_2OH$ | | 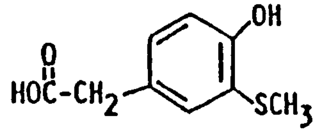 | | 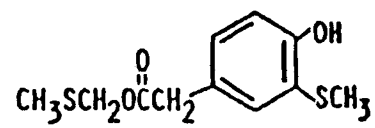 | (1) |
| $CH_3SCH_2OH$ | | 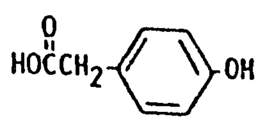 | | 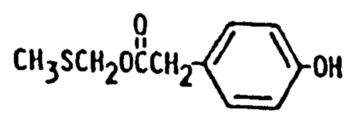 | (2) |

ROH + $HOOC-C_nH_{2n}-Ar-OH$ $ROOC-C_nH_{2n}-Ar-OH$

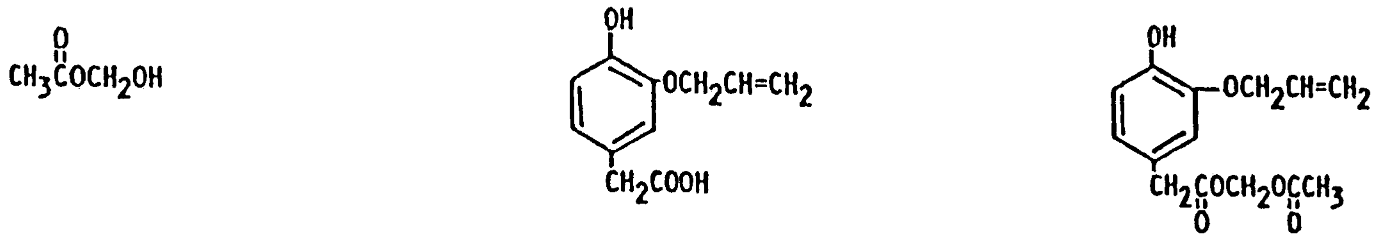

(3)

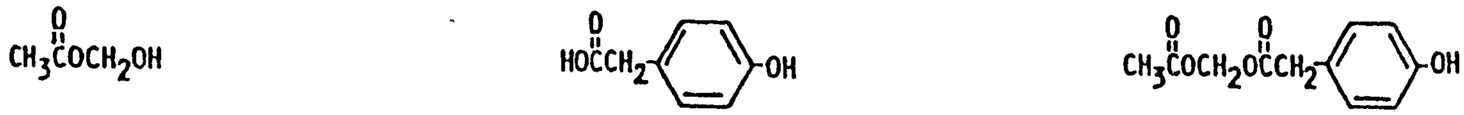

(4)

$CH_3SCH_2OH$

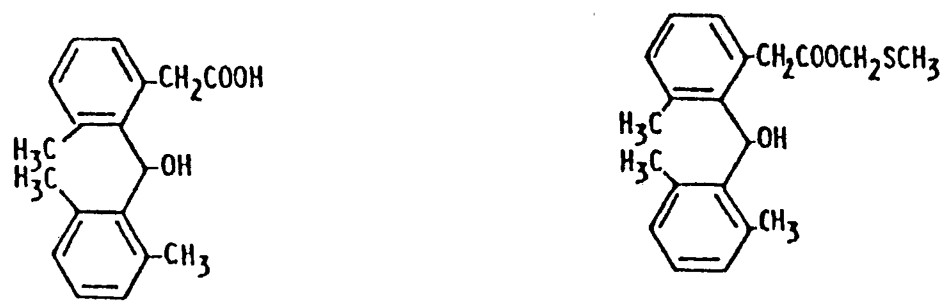

(5)

| ROH | | $HOOC-C_nH_{2n}-Ar-OH$ | | $ROOC-C_nH_{2n}-Ar-OH$ | |
|---|---|---|---|---|---|
| 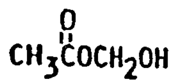 | + | 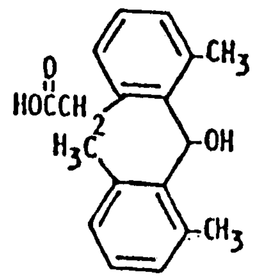 | → | 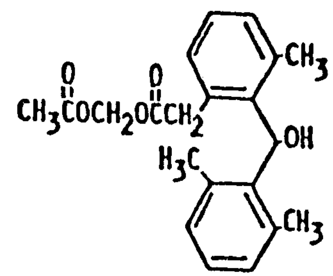 | (6) |
| 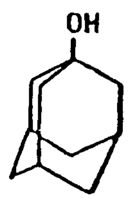 | | 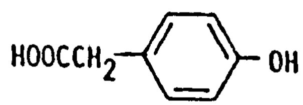 | | 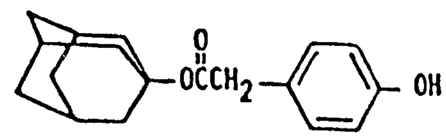 | (7) |
| 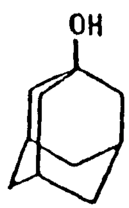 | | 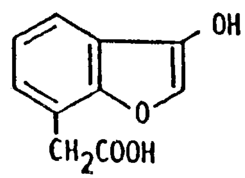 | | 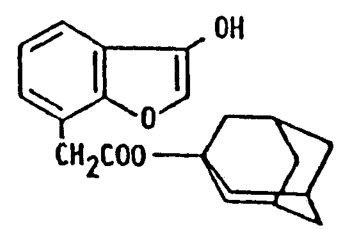 | (8) |

ROH + $HOOC-C_nH_{2n}-A_r-OH$ → $ROOC-C_nH_{2n}-A_r-OH$

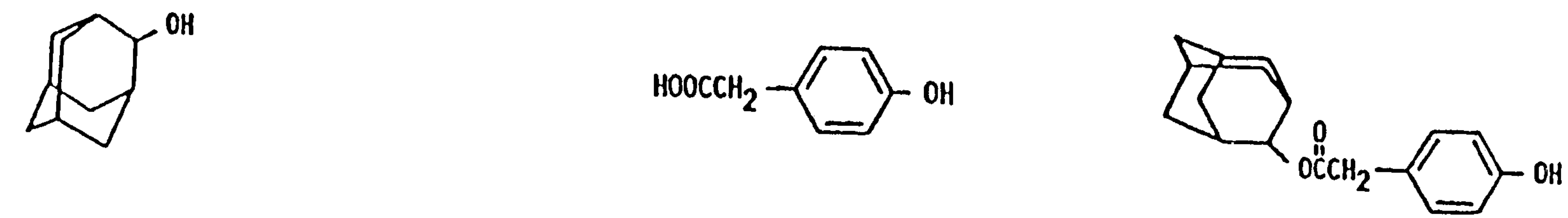

(9)

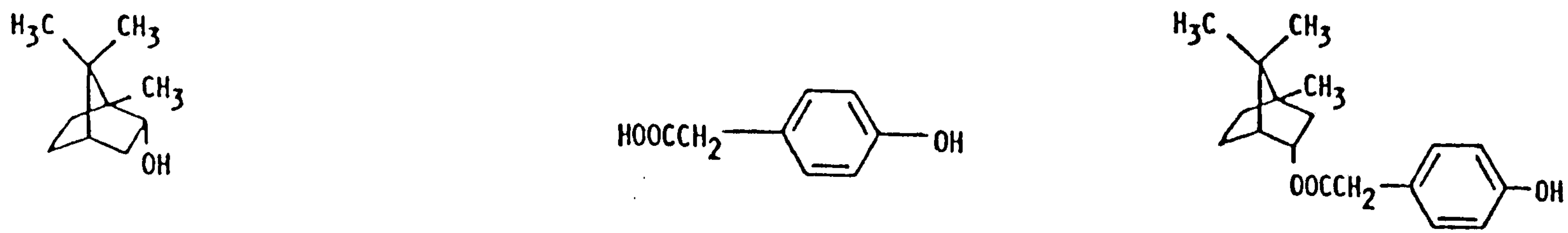

(10)

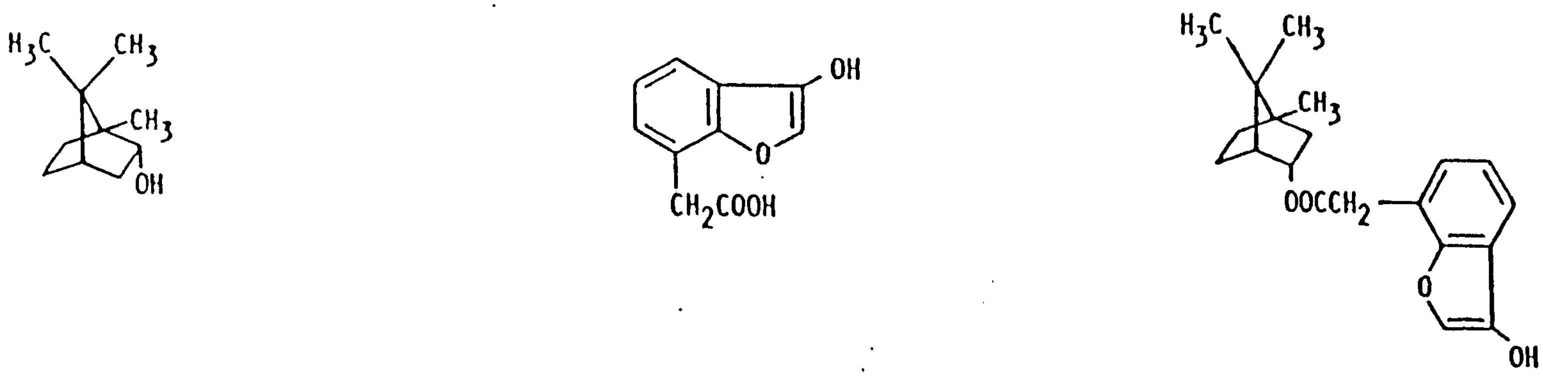

(11)

ROH + HOOC-$C_nH_{2n}$-Ar-OH → ROOC-$C_nH_{2n}$-Ar-OH

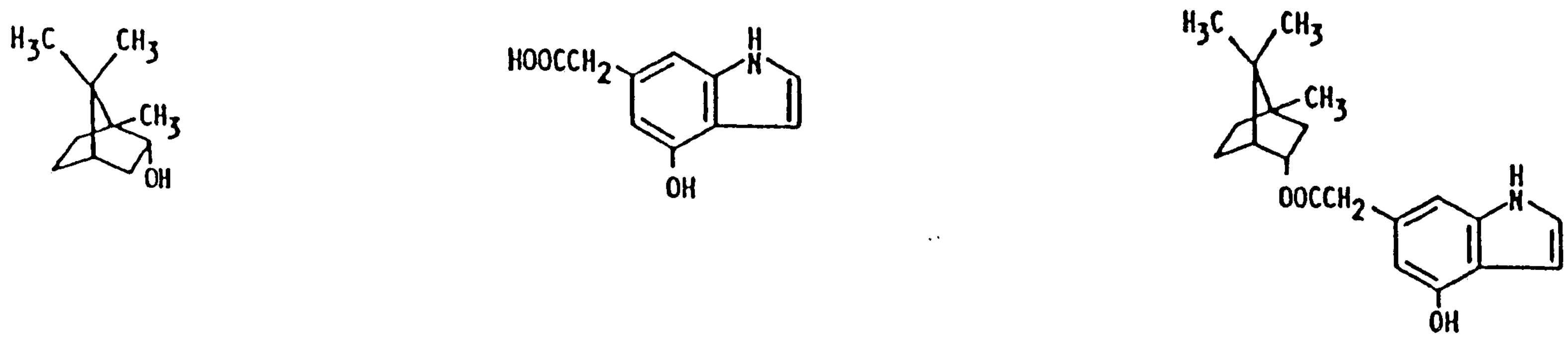

(12)

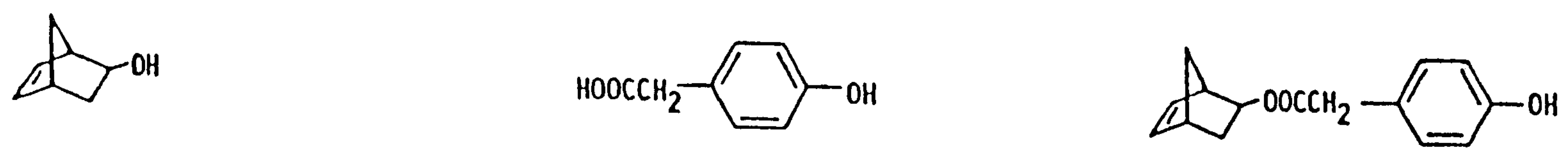

(13)

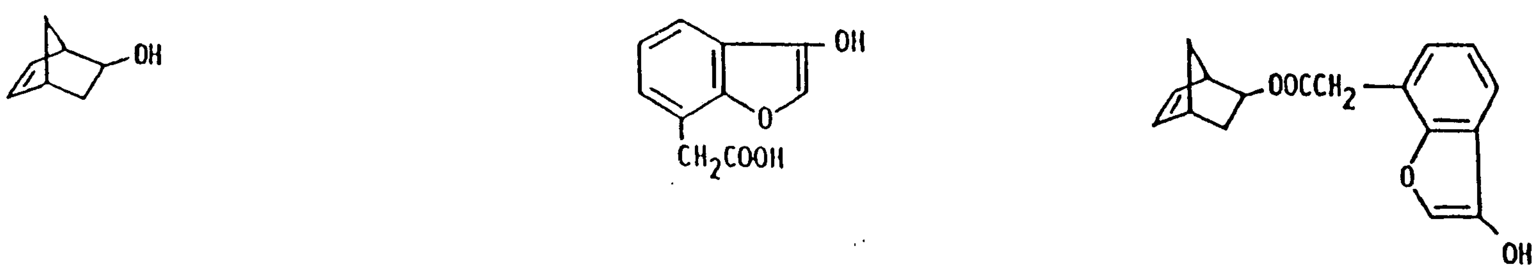

(14)

$$\underline{ROH} \quad + \quad \underline{HOOC\text{-}C_nH_{2n}\text{-}Ar\text{-}OH} \longrightarrow \underline{ROOC\text{-}C_nH_{2n}\text{-}Ar\text{-}OH}$$

| ROH | | HOOC-$C_nH_{2n}$-Ar-OH | ROOC-$C_nH_{2n}$-Ar-OH | |
|---|---|---|---|---|
| 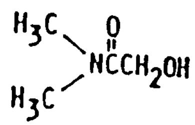 | | 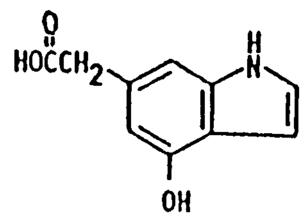 | 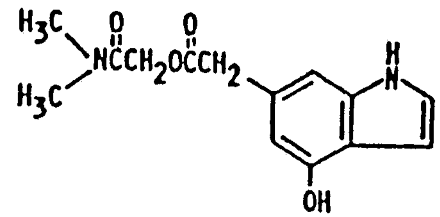 | (15) |
| 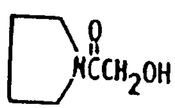 | | 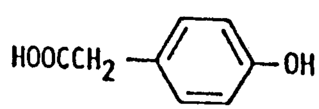 | 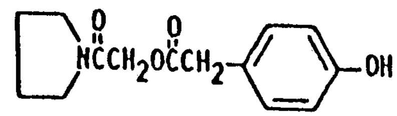 | (16) |

| ROH | + | HOOC-$C_nH_{2n}$-Ar-OH | → | ROOC-$C_nH_{2n}$-Ar-OH | |
|---|---|---|---|---|---|
| $CH_3CH_2SCH_2OH$ | | 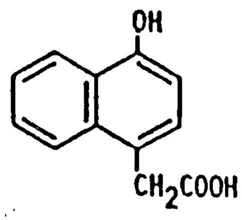 | | 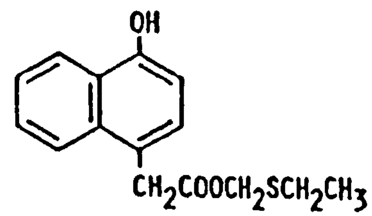 | (17) |
| 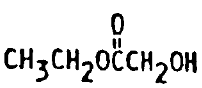 | | 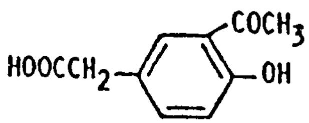 | | 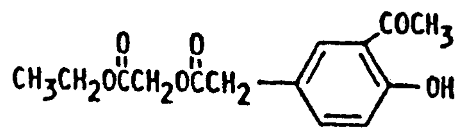 | (18) |

Example 8

Substitution of an equivalent quantity of the products of Example 7 in the general procedures of Example 3, second paragraph [i.e. reaction of a compound of the formula ROOC—$C_nH_{2n}$-Ar-OH with an epihalogenohydrin such as epichlorohydrin, followed by reacting the resultant intermediate(s) with the appropriate primary amine] affords, after appropriate isolation, the following compounds of the invention:

STARTING MATERIAL

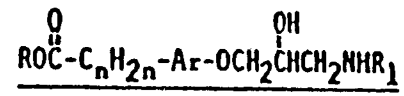

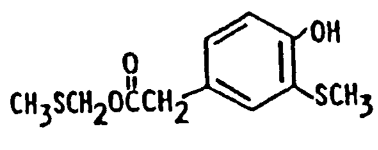

(1)

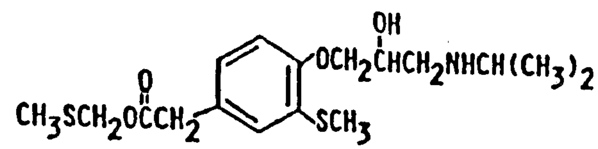

(AN ANALOGUE OF TIPRENOLOL)

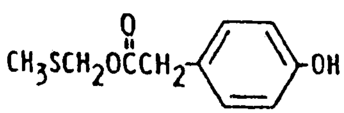

(2)

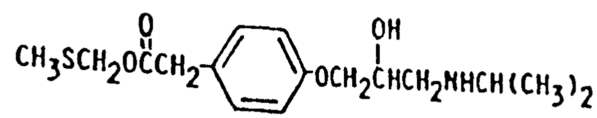

(AN ANALOGUE OF METOPROLOL)

| STARTING MATERIAL | | $RO\overset{O}{\overset{\|}{C}}-C_nH_{2n}-Ar-OCH_2\overset{OH}{\overset{\|}{C}}HCH_2NHR_1$ |
|---|---|---|
| 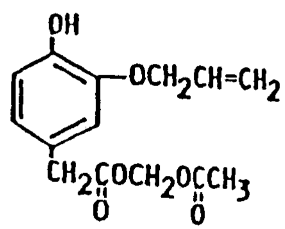 | (3) | 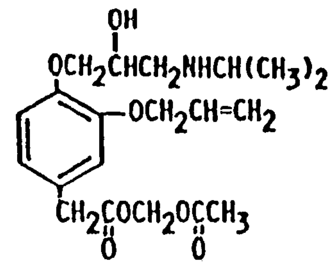<br>(AN ANALOGUE OF OXPRENOLOL) |
| 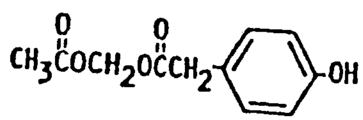 | (4) | 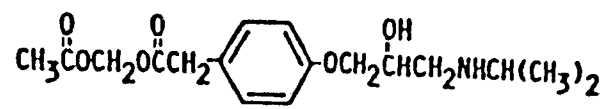<br>(AN ANALOGUE OF METOPROLOL) |
| 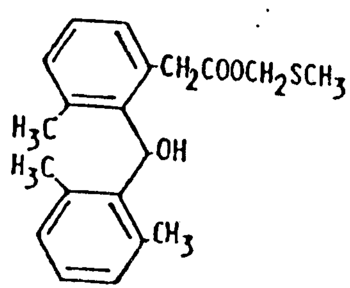 | (5) | 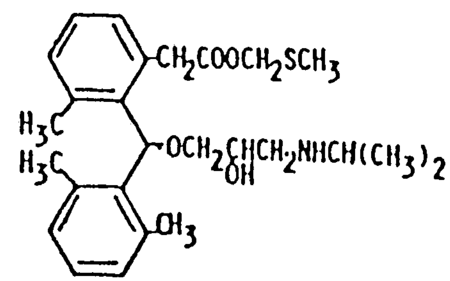<br>( AN ANALOGUE OF XIPRANOLOL) |

STARTING MATERIAL

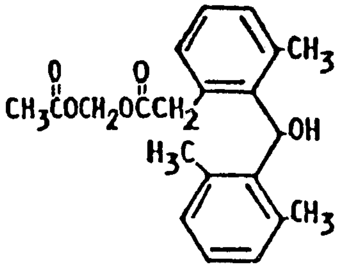

(6)

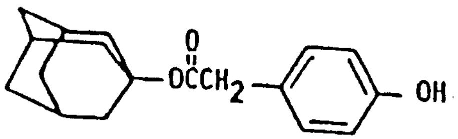

(7)

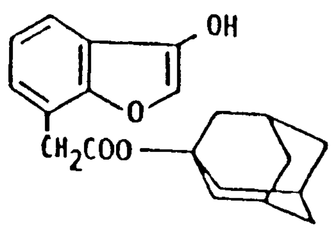

(8)

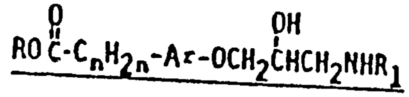

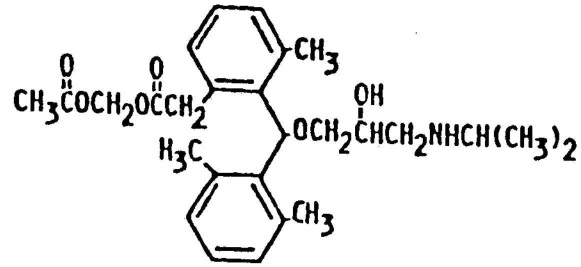

(AN ANALOGUE OF XIPRANOLOL)

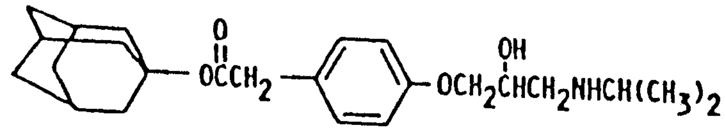

(AN ANALOGUE OF METOPROLOL)

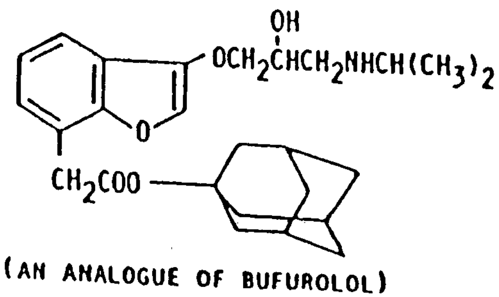

(AN ANALOGUE OF BUFUROLOL)

| STARTING MATERIAL | | 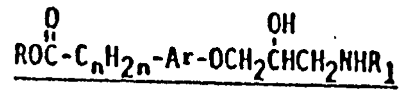 |
|---|---|---|
| 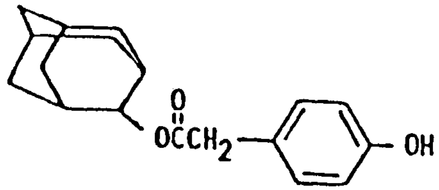 | (9) | 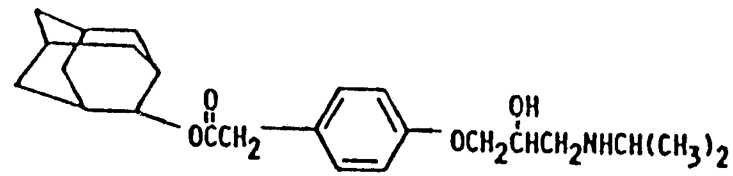<br>(AN ANALOGUE OF METOPROLOL) |
| 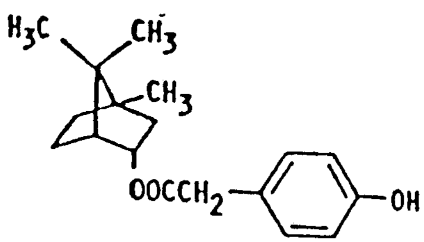 | (10) | 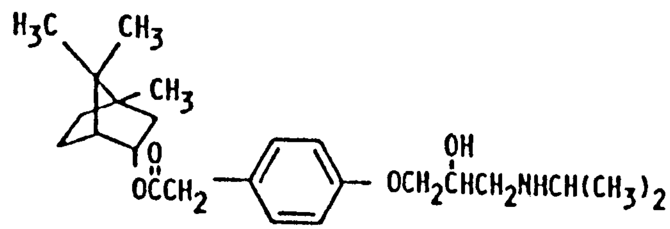<br>(AN ANALOGUE OF METOPROLOL) |
| 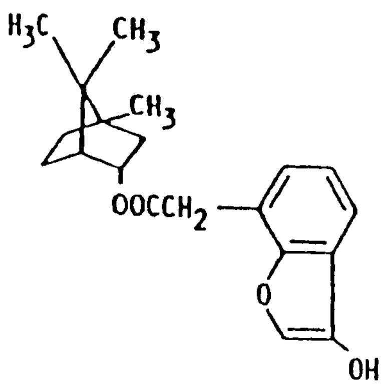 | (11) | 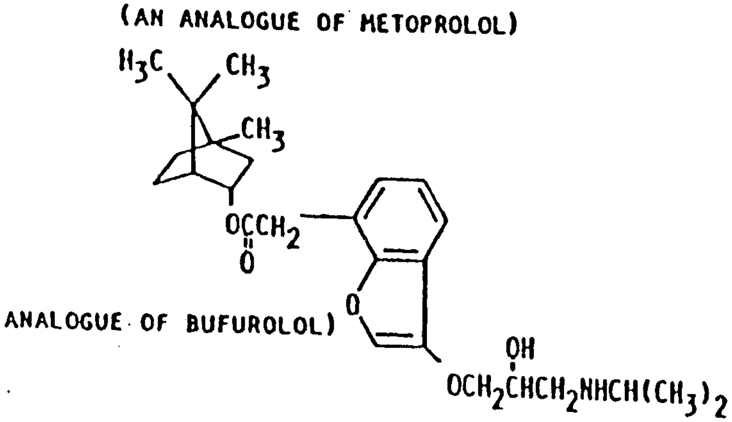<br>(AN ANALOGUE OF BUFUROLOL) |

STARTING MATERIAL

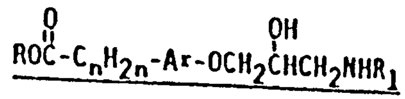

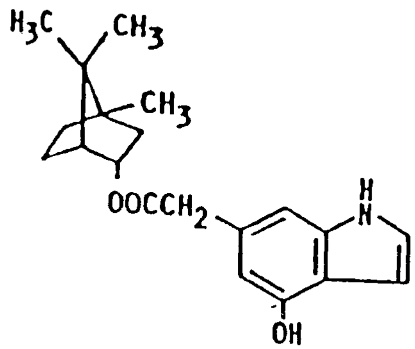

(12)

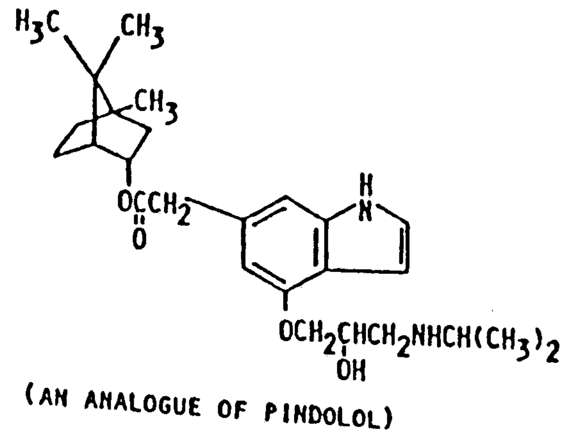

(AN ANALOGUE OF PINDOLOL)

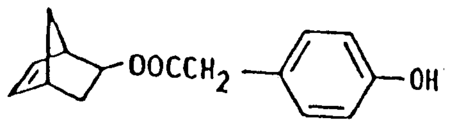

(13)

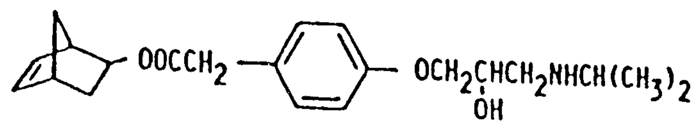

(AN ANALOGUE OF METOPROLOL)

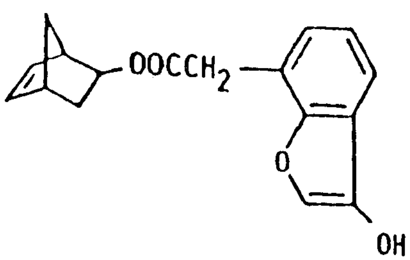

(14)

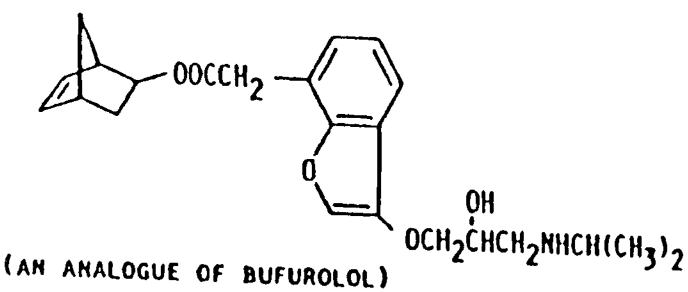

(AN ANALOGUE OF BUFUROLOL)

STARTING MATERIAL

$RO\overset{O}{\overset{\|}{C}}-C_nH_{2n}-Ar-OCH_2\overset{OH}{\overset{|}{C}}HCH_2NHR_1$

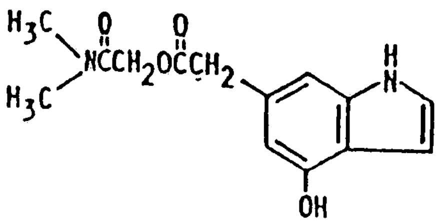

(15)

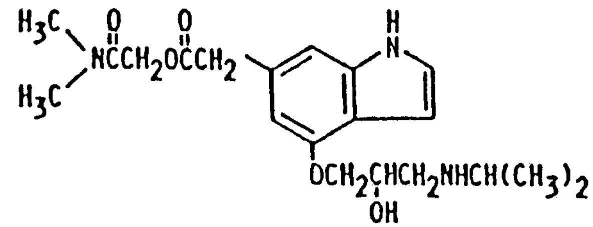

(AN ANALOGUE OF PINDOLOL)

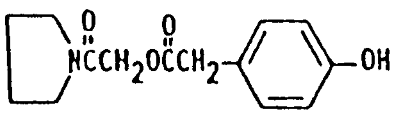

(16)

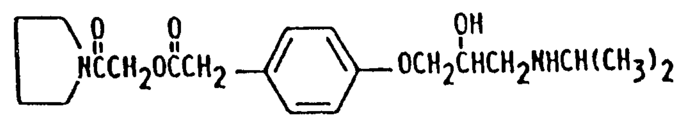

(AN ANALOGUE OF METOPROLOL)

STARTING MATERIAL

$ROC(=O)-C_nH_{2n}-Ar-OCH_2CH(OH)CH_2NHR_1$

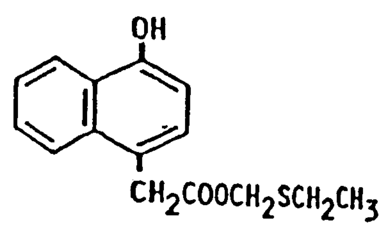

(17)

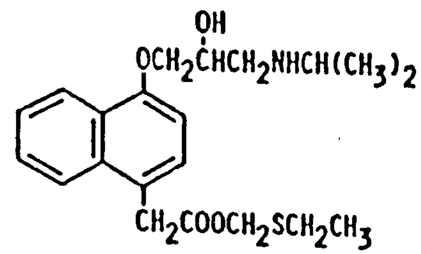

(AN ANALOGUE OF PROPRANOLOL)

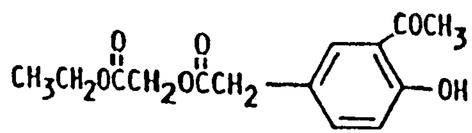

(18)

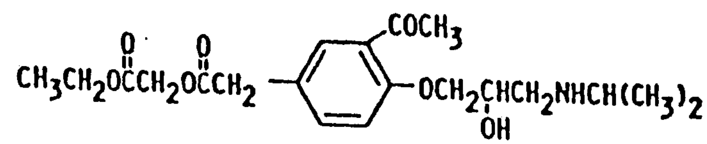

(AN ANALOGUE OF ACEBUTOLOL)

Example 9

Treatment of the sulfur-containing compounds of the invention listed below with one equivalent of *m*-chloroperbenzoic acid affords the corresponding sulfinyl derivatives of the invention, while treatment of the thio compounds with two equivalents of *m*-chloroperbenzoic acid yields the corresponding sulfonyl derivatives of the invention.

| Thio Compound | Sulfinyl Compound | Sulfonyl Compound |
| --- | --- | --- |
| 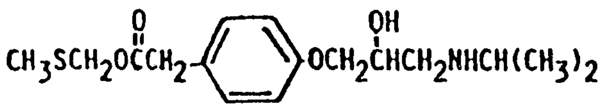 (2) | 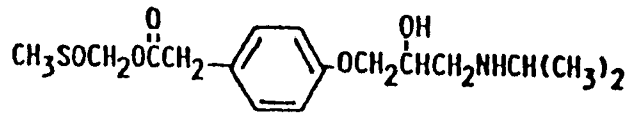 | 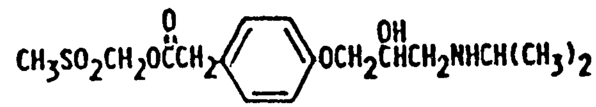 |
| 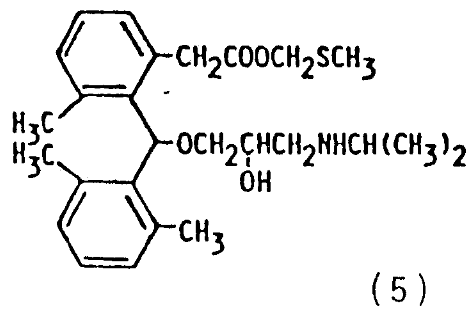 (5) | 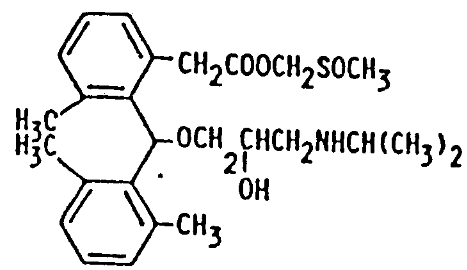 | 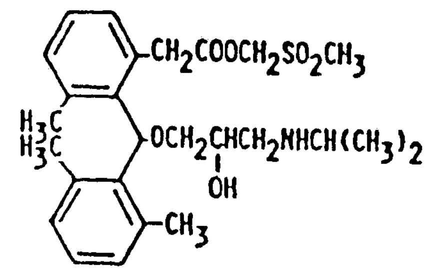 |
| 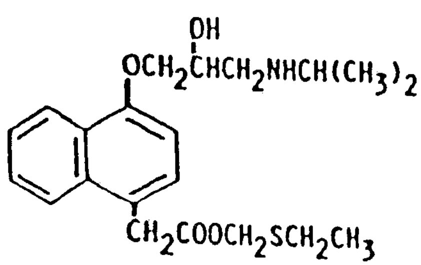 (17) | 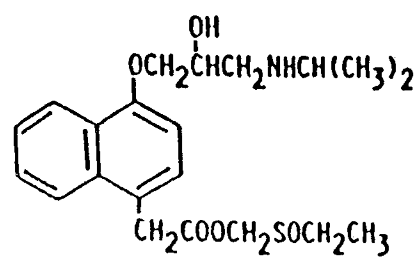 | 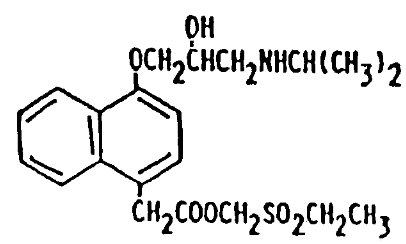 |

**Claims for the Contracting States: BE CH DE FR GB LU NL SE**

1. A compound of the formula

$$RO\overset{\overset{O}{\|}}{C}-C_nH_{2n}-Ar-OCH_2\overset{\overset{OH}{|}}{C}HCH_2NHR_1 \qquad (I)$$

or a pharmaceutically acceptable acid addition salt thereof, wherein:

n is one;

R is $C_6$—$C_{18}$ polycycloalkyl-$C_pH_{2p}$- wherein p is 0, 1, 2, or 3; $C_6$—$C_{18}$ polycycloalkenyl-$C_pH_{2p}$- wherein p is defined as above; $-CH_2-X-R_2$ wherein X is S, SO or $SO_2$ and $R_2$ is $C_1$—$C_7$ alkyl or $C_3$—$C_{12}$ cycloalkyl;

$$-CH_2-\overset{+}{\underset{\underset{CH_3}{|}}{S}}-R_2$$

wherein $R_2$ is defined as above;

$$-\overset{\overset{R_3}{|}}{C}H-X-R_4$$

wherein X is defined as above, and wherein $R_3$ is $C_1$—$C_7$ alkyl and $R_4$ is $C_1$—$C_7$ alkyl or wherein $R_3$ and $R_4$ taken together represent $-(CH_2)_m-$ wherein m is 3 or 4 and $-(CH_2)_m-$ is optionally substituted by one to three $C_1$—$C_7$ alkyl;

$$-\overset{\overset{R_5}{|}}{C}H-OCOR_6$$

wherein $R_5$ is hydrogen or $C_1$—$C_7$ alkyl and $R_6$ is unsubstituted or substituted $C_1$—$C_7$ alkyl, $C_3$—$C_{12}$ cycloalkyl, $C_3$—$C_{12}$ cycloalkenyl or $C_2$—$C_8$ alkenyl, the substituents being selected from the group consisting of halo, $C_1$—$C_7$ alkoxy, $C_1$—$C_7$ alkylthio, $C_1$—$C_7$ alkylsulfinyl, $C_1$—$C_7$ alkylsulfonyl,

$$-NH\overset{\overset{O}{\|}}{C}-(C_1-C_7\ alkyl) \quad and \quad -O\overset{\overset{O}{\|}}{C}-(C_1-C_7\ alkyl),$$

or $R_6$ is substituted or unsubstituted phenyl or benzyl, the substituents being selected from the group consisting of $C_1$—$C_7$ alkyl, $C_1$—$C_7$ alkoxy, halo, carbamoyl, $C_2$—$C_8$ alkoxycarbonyl, $C_2$—$C_8$ alkanoyloxy, $C_1$—$C_7$ haloalkyl, mono($C_1$—$C_7$ alkyl)amino, di($C_1$—$C_7$ alkyl)amino, mono($C_1$—$C_7$ alkyl)carbamoyl, di($C_1$—$C_7$ alkyl)carbamoyl, $C_1$—$C_7$ alkythio, $C_1$—$C_7$ alkylsulfinyl and $C_1$—$C_7$ alkylsulfonyl;

$$\overset{\overset{R_5}{|}}{C}H-COOR_6$$

wherein $R_5$ and $R_6$ are defined as above; or

$$C\overset{\overset{R_5}{|}}{C}H-CONR_7R_8$$

wherein $R_5$ is defined as above and $R_7$ and $R_8$, which can be the same or different, are each hydrogen, $C_1$—$C_7$ alkyl, $C_3$—$C_{12}$ cycloalkyl, phenyl or benzyl, or $R_7$ and $R_8$ are combined such that $-NR_7R_8$ represents the residue of a saturated monocyclic secondary amine;

$R_1$ is $C_1$—$C_7$ alkyl; and

Ar is a phenylene radical which is unsubstituted or which is substituted by $C_1$—$C_7$ alkyl, $C_1$—$C_7$ alkyl—O—$C_1$—$C_7$ alkylene-, $C_2$—$C_8$ alkenyl, $C_1$—$C_7$ alkyl—S—, $C_2$—$C_8$ alkenyl—O—, $C_3$—$C_{12}$ cycloalkyl, $C_1$—$C_7$ alkyl—CONH—, $C_1$—$C_7$ alkyl—CO— or $H_2NCO$—$C_1$—$C_7$ alkylene-; or

Ar is a radical of the formula

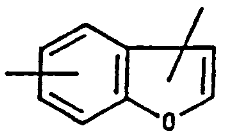, 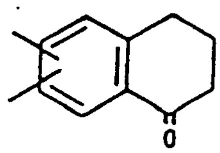, 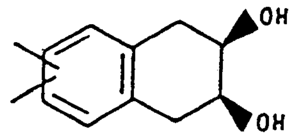, 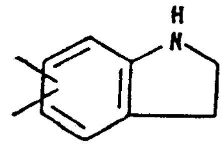,

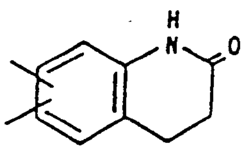, 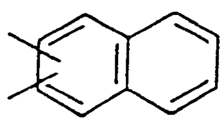 or 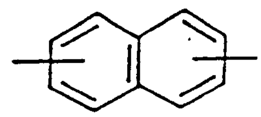.

or Ar is a phenylene-$C_1$—$C_3$ alkylene-phenyl radical.

2. A compound as defined by Claim 1, wherein R is $C_6$—$C_{18}$ polycycloalkyl—$C_pH_{2p}$— consisting of a two or three ring bridged saturated alicyclic hydrocarbon system bearing zero, one or two methyl substituents and having a total of 6 to 18 carbon atoms in the polycycloalkyl portion and p is zero, one or two.

3. A compound as defined by Claim 1, wherein R is $C_6$—$C_{18}$ polycycloalkenyl—$C_pH_{2p}$— consisting of a two or three ring bridged unsaturated alicyclic hydrocarbon system bearing zero, one or two methyl substituents and having a total of 6 to 18 carbon atoms in the polycycloalkenyl portion and p is zero, one or two.

4. A compound as defined by Claim 1, wherein R is adamantyl, bornyl, norbornyl, adamantylmethyl, adamantylethyl, nobornylmethyl, borbornylethyl or norbornenyl.

5. A compound as defined by Claim 1, wherein R is (adamant-1-yl)ethyl, (adamant-1-yl)methyl or (norborn-2-yl)methyl.

6. A compound as defined by Claim 1, wherein R is

$$\overset{R_5}{\overset{|}{—CH}}—OCOR_6 \text{ or } \overset{R_5}{\overset{|}{—CH}}—OCOR_7R_8,$$

wherein $R_5$, $R_6$, $R_7$ and $R_8$ are as defined in Claim 1.

7. A compound as defined by Claim 6, wherein $R_5$ is hydrogen and $R_6$ is $C_1$—$C_7$ alkyl.

8. A compound as defined by Claim 6, wherein R is is pivalyloxymethyl or carbamoylmethyl.

9. A compound as defined by any one of Claims 1 to 8, wherein Ar comprises a phenylene grouping.

10. A compound as defined by Claim 9, wherein said phenylene grouping is unsubstituted phenylene.

11. A compound as defined by Claim 10, wherein the unsubstituted phenylene groupins 1,4-phenylene or 1,3-phenylene.

12. A compound as defined by any one of Claims 1 to 8 , wherein Ar is phenylene substituted by $C_1$—$C_7$ alkyl, $C_1$—$C_7$ alkyl-O—$C_1$—$C_7$ alkylene-, $C_2$—$C_8$ alkenyl, $C_1$—$C_7$ alkyl-S—, $C_2$—$C_8$ alkenyl-O—, $C_3$—$C_{12}$ cycloalkyl, $C_1$—$C_7$ alkyl-CONH—, $C_1$—$C_7$ alkyl-CO— or $H_2NCO$-$C_1$—$C_7$ alkylene-.

13. A compound as defined by Claim 12, wherein Ar is phenylene bearing a $CH_3CH_2$—, $CH_3$—, $CH_3OCH_2CH_2$—, $CH_2$=CH—$CH_2$—, $CH_3$—S—, $CH_2CH$=$CH_2$—O—,

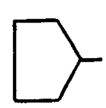,

$CH_3CH_2CH_2CONH$—, $CH_3CONH$—, $CH_3CO$— or $H_2NCO$—$CH_2$—.

14. A compound as defined by any one of Claims 1 to 8, wherein Ar is a radical of the formula

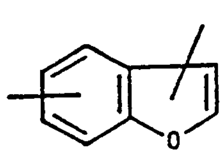, 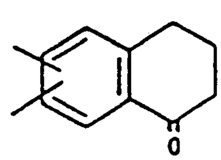, 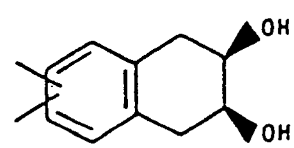, 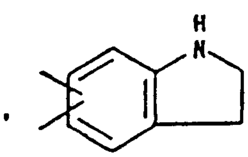,

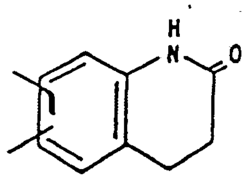, 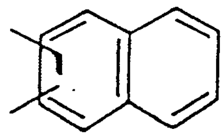 or 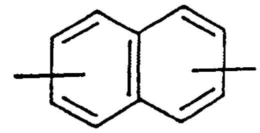.

15. A compound as defined by Claim 14, wherein Ar is a radical of the formula

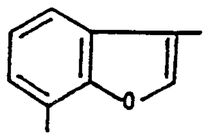, 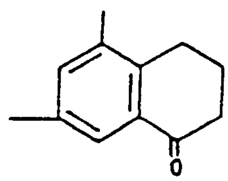, 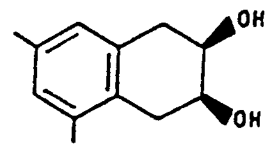,

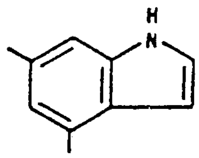, 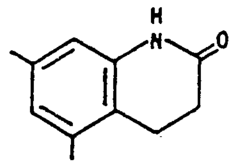 or 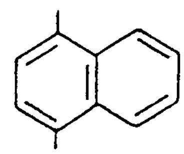.

16. A compound as defined by any one of Claim 1 to 8, wherein Ar is a phenylene-$C_1$—$C_3$ alkylene-phenyl radical.

17. A compound as defined by Claim 16, wherein Ar is a radical of the formula

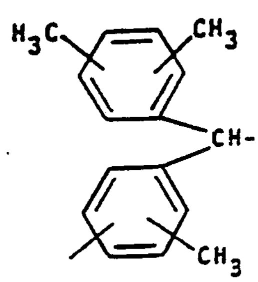

18. A compound as defined by Claim 1, wherein the

$$RO\overset{\overset{O}{\|}}{C}—C_nH_{2n}—$$

grouping replaces a hydrogen atom on the corresponding ring of a known β-blocker, the structure of the compound of formula (I) being in all other respects identical to the structure of a known β-blocker.

19. A compound as defined by Claim 18, wherein the known β-blocker is metoprolol, bufurolol, alprenolol, bunolol, xipranolol, nadolol, tiprenolol, oxprenolol, penbutolol, pindolol, carteolol, propranolol, acebutolol, atenolol or practolol.

20. A compound as defined by Claim 1, wherein the

$$RO\overset{\overset{O}{\|}}{C}—C_nH_{2n}—$$

grouping replaces a non-critical substituent on the corresponding ring of a known β-blocker, the structure of the compound of formula (I) being in all other respects identical to the structure of a known β-blocker.

21. A compound as defined by Claim 21, wherein the non-critical substituent is a lower alkyl or lower alkyl-O-lower alkylene substituent.

22. A compound as defined by Claim 21, wherein the non-critical substituent is a $CH_3$—, $CH_3CH_2$— or $CH_3OCH_2CH_2$— substituent.

23. A compound as defined by Claim 20, wherein the known β-blocker is metoprolol, bufurolol or xipranolol.

24. A compound as defined by Claim 20, wherein the non-critical substituent is a lower alkyl-CONH— or $H_2NCO$— lower alkylene- substituent.

25. A compound as defined by Claim 24, wherein the non-critical substituent is a $CH_3CH_2CH_2CONH$—, $CH_3CONH$— or

$$H_2N\overset{O}{\overset{\|}{C}}CH_2\text{— substituent.}$$

26. A compound as defined by Claim 20, wherein the known β-blocker is acebutolol, atenolol or practolol.

27. A compound as defined by any one of Claims 1 to 17, wherein $R_1$ is isopropyl or tert-butyl.

28. A compound as defined by Claim 1, which is (adamant-1-yl)ethyl 4-(2-hydroxy-3-isopropylamino)-propoxyphenylacetate or a pharmaceutically acceptable acid addition salt thereof.

29. A compound as defined by Claim 1, which is (adamant-1-yl)methyl 4-(2-hydroxy-3-isopropylamino)-propoxyphenylacetate or a pharmaceutically acceptable acid addition salt thereof.

30. A compound as defined by Claim 1, which is (noborn-2-yl)methyl 4-(2-hydroxy-3-isopropylamino)-propoxyphenylacetate or a pharmaceutically acceptable acid addition salt thereof.

31. A compound as defined by Claim 1, which is pivalyloxymethyl 4-(2-hydroxy-3-isopropylamino)propoxyphenylacetate or a pharmaceutically acceptable acid addition salt thereof.

32. A compound as defined by Claim 1, which is carbamoylmethyl 4-(2-hydroxy-3-isopropylamino)propoxyphenylacetate or a pharmaceutically acceptable acid addition salt thereof.

33. An oxalate salt of a compound as defined by any one of Claims 1 to 32.

34. A pharmaceutical composition of matter comprising a compound of formula (I) as defined by any one of the preceding Claims, or a pharmaceutically acceptable acid addition salt thereof, and a non-toxic pharmaceutically acceptable carrier therefor.

35. A pharmaceutical composition of matter, in unit dosage form, for use in eliciting a β-adrenergic blocking response in a warm-blooded animal, said composition comprising, per dosage unit, an effective unit β-ardrenergic blocking amount of a compound of formula (I) as defined by any one of the preceding Claims, or a pharmaceutically acceptable acid addition salt thereof, and a non-toxic pharmaceutically acceptable carrier therefor.

36. An ophthalmic composition of matter comprising a compound of formula (I) as defined by any one of the preceding Claims, or a pharmaceutically acceptable acid addition salt thereof, and a non-toxic ophthalmically acceptable carrier therefor.

37. An ophthalmic composition of matter, in unit dosage form, for use in the treatment of glaucoma in a warm-blooded animal, said composition comprising, per dosage unit, an effective unit intraocular pressure decreasing amount of a compound of formula (I) as defined by any one of the preceding Claims or a pharmaceutically acceptable acid addition salt thereof, and a non-toxic ophthalmically acceptable carrier therefor.

**Claims for the Contracting State: AT**

1. A process for the preparation of a compound of the formula

$$RO\overset{O}{\overset{\|}{C}}-C_nH_{2n}-Ar-OCH_2\overset{OH}{\overset{|}{C}}HCH_2NHR_1 \qquad (I)$$

or a pharmaceutical acceptable acid addition salt thereof, wherein:

n is one;

R is $C_6$—$C_{18}$ polycycloalkyl-$C_pH_{2p}$— wherein p is 0, 1, 2, or 3; $C_6$—$C_{18}$ polycycloalkenyl-$C_pH_{2p}$— wherein p is defined as above; —$CH_2$—X—$R_2$ wherein X is S, SO or $SO_2$ and $R_2$ is $C_1$—$C_7$ alkyl or $C_3$—$C_{12}$ cycloalkyl;

$$-CH_2-\overset{+}{S}(CH_3)-R_2$$

wherein $R_2$ is defined as above;

$$-\overset{R_3}{\overset{|}{C}}H-X-R_4$$

wherein X is defined as above, and wherein $R_3$ is $C_1$—$C_7$ alkyl and $R_4$ is $C_1$—$C_7$ alkyl or wherein $R_3$ and $R_4$ taken together represent —$(CH_2)_m$— wherein m is 3 or 4 and —$(CH_2)_m$— is optionally substituted by one to three $C_1$—$C_7$ alkyl;

$$\overset{R_5}{\overset{|}{-CH}}-OCOR_6$$

wherein $R_5$ is hydrogen or $C_1$—$C_7$ alkyl and $R_6$ is unsubstituted or substituted $C_1$—$C_7$ alkyl, $C_3$—$C_{12}$ cycloalkyl, $C_3$—$C_{12}$ cycloalkenyl or $C_2$—$C_8$ alkenyl, the substituents being selected from the group consisting of halo, $C_1$—$C_7$ alkoxy, $C_1$—$C_7$ alkylthio, $C_1$—$C_7$ alkylsulfinyl, $C_1$—$C_7$ alkylsulfonyl,

$$-NH\overset{O}{\overset{\|}{C}}-(C_1-C_7\text{ alkyl})\quad\text{and}\quad -O\overset{O}{\overset{\|}{C}}-(C_1-C_7\text{ alkyl}),$$

or $R_6$ is substituted or unsubstituted phenyl or benzyl, the substituents being selected from the group consisting of $C_1$—$C_7$ alkyl, $C_1$—$C_7$ alkoxy, halo, carbamoyl, $C_2$—$C_8$ alkoxycarbonyl, $C_2$—$C_8$ alkanoyloxy, $C_1$—$C_7$ haloalkyl, mono($C_1$—$C_7$ alkyl)amino, di($C_1$—$C_7$ alkyl)amino, mono($C_1$—$C_7$ alkyl)carbamoyl, di($C_1$—$C_7$ alkyl)carbamoyl, $C_1$—$C_7$ alkythio, $C_1$—$C_7$ alkylsulfinyl and $C_1$—$C_7$ alkylsulfonyl;

$$\overset{R_5}{\overset{|}{-CH}}-COOR_6$$

wherein $R_5$ and $R_6$ are defined as above; or

$$\overset{R_5}{\overset{|}{CH}}-CONR_7R_8$$

wherein $R_5$ is defined as above and $R_7$ and $R_8$, which can be the same or different, are each hydrogen, $C_1$—$C_7$ alkyl, $C_3$—$C_{12}$ cycloalkyl, phenyl or benzyl, or $R_7$ and $R_8$ are combined such that —$NR_7R_8$ represents the residue of a saturated monocyclic secondary amine;

$R_1$ is $C_1$—$C_7$ alkyl; and

Ar is a phenylene radical which is unsubstituted or which is substituted by $C_1$—$C_7$ alkyl, $C_1$—$C_7$ alkyl—O—$C_1$—$C_7$ alkylene-, $C_2$—$C_8$ alkenyl, $C_1$—$C_7$ alkyl—S—, $C_2$—$C_8$ alkenyl—O—, $C_3$—$C_{12}$ cycloalkyl, $C_1$—$C_7$ alkyl—CONH—, $C_1$—$C_7$ alkyl—CO— or $H_2NCO$—$C_1$—$C_7$ alkylene-; or

Ar is a radical of the formula

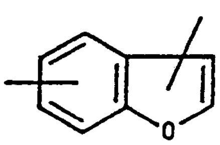, 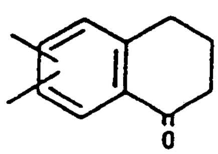, 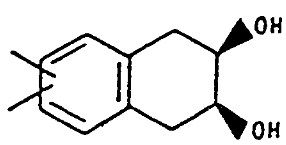, 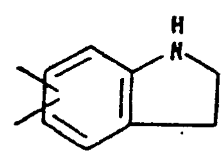,

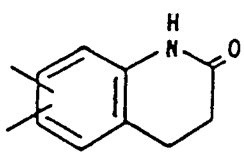, 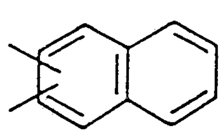 or 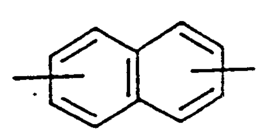.

or Ar is a phenylene-$C_1$—$C_3$ alkylene-phenyl radical; said process comprising:

(a) reacting the corresponding compound of the formula

$$RO\overset{O}{\overset{\|}{C}}-C_nH_{2n}-Ar-OCH_2-Z \qquad (II)$$

wherein R, n and Ar are defined as above and Z is

$$-\overset{O}{\overset{/\backslash}{CH-CH_2}}\ \text{or}\ -\overset{OH}{\overset{|}{C}}HCH_2-Hal$$

wherein Hal is a halogen atom, with a primary amine of the formula

$NH_2R_1$

wherein $R_1$ is defined as above; or

(b) esterifying the corresponding acid of the formula

$$HOOC-C_nH_{2n}-Ar-OCH_2\overset{\overset{OH}{|}}{C}HCH_2NHR_1 \qquad (X)$$

wherein n, Ar and $R_1$ are defined as above, or the corresponding acid chloride or acid anhydride, by reaction with an alcohol of the formula

$$ROH \qquad (VII)$$

wherein R is defined as above; or

(c) when a compound of formula (I) containing a sulfinyl or sulfonyl group is desired, oxidizing the corresponding sulfur-containing compound of formula (I); or

(d) when a pharmaceutically acceptable acid addition salt is desired, reacting the corresponding compound of formula (I) with a pharmaceutically acceptable inorganic or organic acid.

2. A process as defined by Claim 1, wherein R is $C_6$—$C_{18}$ polycycloalkyl—$C_pH_{2p}$— consisting of a two or three ring bridged saturated alicyclic hydrocarbon system bearing zero, one or two methyl substituents and having a total of 6 to 18 carbon atoms in the polycycloalkyl portion and p is zero, one or two.

3. A process as defined by Claim 1, wherein R is $C_6$—$C_{18}$ polycycloalkenyl—$C_pH_{2p}$— consisting of a two or three ring bridged unsaturated alicyclic hydrocarbon system bearing zero, one or two methyl substituents and having a total of 6 to 18 carbon atoms in the polycycloalkenyl portion and p is zero, one or two.

4. A process as defined by Claim 1, wherein R is adamantyl, bornyl, norbornyl, adamantylmethyl, adamantylethyl, nobornylmethyl, borbornylethyl or norbornenyl.

5. A process as defined by Claim 1, wherein R is (adamant-1-yl)ethyl, (adamant-1-yl)methyl or (norborn-2-yl)methyl.

6. A process as defined by Claim 1, wherein R is

$$-\overset{\overset{R_5}{|}}{C}H-OCOR_6 \text{ or } -\overset{\overset{R_5}{|}}{C}H-OCOR_7R_8,$$

wherein $R_5$, $R_6$, $R_7$ and $R_8$ are as defined in Claim 1.

7. A process as defined by Claim 6, wherein $R_5$ is hydrogen and $R_6$ is $C_1$—$C_7$ alkyl.

8. A process as defined by Claim 6, wherein R is pivalyloxymethyl or carbamoylmethyl.

9. A process as defined by any of Claims 1 to 8, wherein Ar comprises a phenylene grouping.

10. A process as defined by Claim 9, wherein said phenylene grouping is unsubstituted phenylene.

11. A process as defined by Claim 10, wherein the unsubstituted phenylene groupings 1,4-phenylene or 1,3-phenylene.

12. A process as defined by any one of Claims 1 to 8 , wherein Ar is phenylene substituted by $C_1$—$C_7$ alkyl, $C_1$—$C_7$ alkyl-O—$C_1$—$C_7$ alkylene-, $C_2$—$C_8$ alkenyl, $C_1$—$C_7$ alkyl-S—, $C_2$—$C_8$ alkenyl-O—, $C_3$—$C_{12}$ cycloalkyl, $C_1$—$C_7$ alkyl-CONH—, $C_1$—$C_7$ alkyl-CO— or $H_2NCO$-$C_1$—$C_7$ alkylene-.

13. A process as defined by Claim 12, wherein Ar is phenylene bearing a $CH_3CH_2$—, $CH_3$—, $CH_3OCH_2CH_2$—, $CH_2$=CH—$CH_2$—, $CH_3$—S—, $CH_2CH$=$CH_2$—O—,

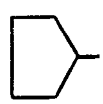 ,

$CH_3CH_2CH_2CONH$—, $CH_3CONH$—, $CH_3CO$— or $H_2NCO$—$CH_2$—.

14. A process as defined by any one of Claims 1 to 8, wherein Ar is a radical of the formula

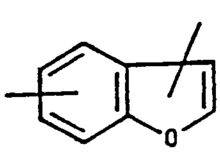 , 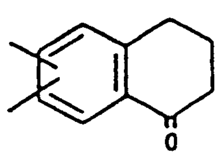 , 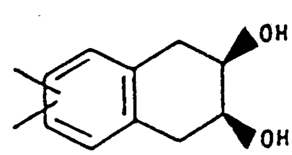 , 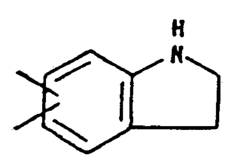 ,

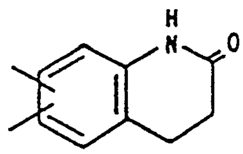, 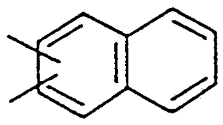 or 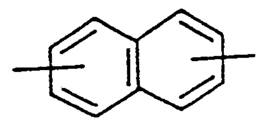.

15. A process as defined by Claim 14, wherein Ar is a radical of the formula

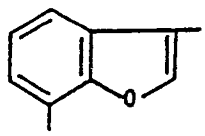, 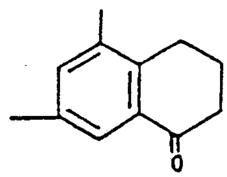, 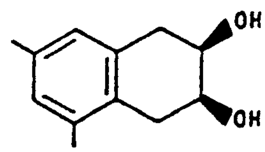,

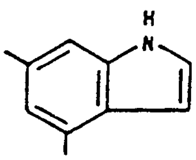, 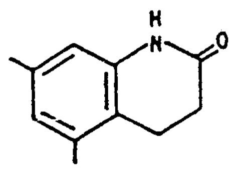 or 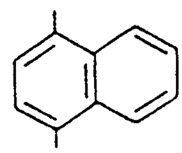.

16. A process as defined by any one of Claim 1 to 8, wherein Ar is a phenylene-$C_1$—$C_3$ alkylene-phenyl radical.

17. A process as defined by Claim 16, wherein Ar is a radical of the formula

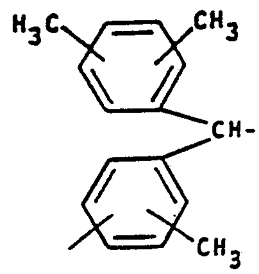

18. A process as defined by Claim 1, wherein the

$$RO\overset{O}{\overset{\|}{C}}—C_nH_{2n}—$$

grouping replaces a hydrogen atom on the corresponding ring of a known β-blocker, the structure of the compound of formula (I) being in all other respects identical to the structure of a known β-blocker.

19. A process as defined by Claim 18, wherein the known β-blocker is metoprolol, bufurlol, alprenolol, bunolol, xipranolol, nadolol, tiprenolol, oxprenolol, penbutolol, pindolol, carteolol, propranolol, acebutolol, atenolol or practolol.

20. A process as defined by Claim 1, wherein the

$$RO\overset{O}{\overset{\|}{C}}—C_nH_{2n}—$$

grouping replaces a non-critical substituent on the corresponding ring of a known β-blocker, the structure of the compound of formula (I) being in all other respects identical to the structure of a known β-blocker.

21. A process as defined by Claim 20, wherein the non-critical substituent is a lower alkyl or lower alkyl-O-lower alkylene substituent.

22. A process as defined by Claim 21, wherein the non-critical substituent is a $CH_3$—, $CH_3CH_2$— or $CH_3OCH_2CH_2$— substituent.

23. A process as defined by Claim 20, wherein the known β-blocker is metoprolol, bufurolol or xipranolol.

24. A process as defined by Claim 20, wherein the non-critical substituent is a lower alkyl-CONH— or

$H_2NCO$— lower alkylene- substituent.

25. A process as defined by Claim 24, wherein the non-critical substituent is a $CH_3CH_2CH_2CONH$—, $CH_3CONH$— or

O
‖
$H_2NCCH_2$— substituent.

26. A process as defined by Claim 20, wherein the known β-blocker is acebutolol, atenolol or practolol.

27. A process as defined by any one of Claims 1 to 17, wherein $R_1$ is isopropyl or tert-butyl.

28. A process as defined by Claim 1, wherein the compound of formula (I) is (adamant-1-yl)ethyl 4-(2-hydroxy-3-isopropylamino)-propoxyphenylacetate or a pharmaceutically acceptable acid addition salt thereof.

29. A process as defined by Claim 1, wherein the compound of formula (I) is (adamant-1-yl)methyl 4-(2-hydroxy-3-isopropylamino)-propoxyphenylacetate or a pharmaceutically acceptable acid addition salt thereof.

30. A process as defined by Claim 1, wherein the compound of formula (I) is (norborn-2-yl)methyl 4-(2-hydroxy-3-isopropylamino)-propoxyphenylacetate or a pharmaceutically acceptable acid addition salt thereof.

31. A process as defined by Claim 1, wherein the compound of formula (I) is pivalyloxymethyl 4-(2-hydroxy-3-isopropylamino)propoxyphenylacetate or a pharmaceutically acceptable acid addition salt thereof.

32. A process as defined by Claim 1, wherein the compound of formula (I) is carbamoylmethyl 4-(2-hydroxy-3-isopropylamino)propoxyphenylacetate or a pharmaceutically acceptable acid addition salt thereof.

33. A process according to any one of Claims 1 to 32, wherein the compound of formula (I) is reacted with oxalic acid to afford the corresponding oxalate salt.

**Patentansprüche für die Vertragsstaaten: BE CH DE FR GB LU NL SE**

1. Verbindung der Formel

O ‖ (on C), OH | (on CH)
$ROC—C_nH_{2n}—Ar—OCH_2CHCH_2NHR_1$ (I)

oder ein pharmazeutisch verträgliches Säureadditionssalz davon, worin

n 1 ist;

R $C_6$—$C_{18}$-Polycycloalkyl—$C_pH_{2p}$— ist, worin p 0, 1, 2 oder 3 ist, $C_6$—$C_{18}$-Polycycloalkenyl—$C_pH_{2p}$—, worin p die genannten Bedeutungen hat; —$CH_2$—X—$R_2$, worin X S, SO oder $SO_2$ ist und $R_2$ $C_1$—$C_7$-Alkyl oder $C_3$—$C_{12}$-Cycloalkyl ist;

+
—$CH_2$—S—$R_2$,
|
$CH_3$

worin $R_2$ die oben genannten Bedeutungen hat;

$R_3$
|
—CH—X—$R_4$,

worin X die oben genannten Bedeutungen hat, und worin $R_3$ $C_1$—$C_7$-Alkyl und $R_4$ $C_1$—$C_7$-Alkyl bedeutet, oder worin $R_3$ und $R_4$ zusammengenommen —$(CH_2)_m$— bedeuten, worin m 3 oder 4 ist und —$(CH_2)_m$— gegebenenfalls durch 1 bis 3 $C_1$—$C_7$-Alkyle substituiert ist;

$R_5$
|
—CH—$OCOR_6$,

worin $R_5$ H oder $C_1$—$C_7$-Alkyl ist und $R_6$ gegebenenfalls substituiertes $C_1$—$C_7$-Alkyl, $C_3$—$C_{12}$-Cycloalkyl, $C_3$—$C_{12}$-Cycloalkenyl oder $C_2$—$C_8$-Alkenyl ist, wobei die Substituenten ausgewählt sind aus der Gruppe, bestehend aus Halogen, $C_1$—$C_7$-Alkoxy, $C_1$—$C_7$-Alkylthio, $C_1$—$C_7$-Alkylsulfinyl, $C_1$—$C_7$-Alkylsulfonyl,

$$-NH\overset{\overset{\large O}{\|}}{C}-(C_1-C_7\text{-Alkyl}) \quad \text{und} \quad -O\overset{\overset{\large O}{\|}}{C}-(C_1-C_7\text{-Alkyl}),$$

oder $R_6$ gegebenenfalls substituiertes Phenyl oder Benzyl ist, wobei die Substituenten ausgewählt sind aus der Gruppe, bestehend aus $C_1$—$C_7$-Alkyl, $C_1$—$C_7$-Alkoxy, Halogen, Carbamoyl, $C_2$—$C_8$-Alkoxycarbonyl, $C_2$—$C_8$-Alkanoyloxy, $C_1$—$C_7$-Haloalkyl, Mono($C_1$—$C_7$-alkyl)amino, Di($C_1$—$C_7$-alkyl)amino, Mono($C_1$—$C_7$-alkyl)carbamoyl, Di($C_1$—$C_7$-alkyl)carbamoyl, $C_1$—$C_7$-Alkylthio, $C_1$—$C_7$-Alkylsulfinyl und $C_1$—$C_7$-Alkylsulfonyl;

$$-\overset{\overset{\large R_5}{|}}{C}H-COOR_6,$$

worin $R_5$ und $R_6$ die genannten Bedeutungen haben, oder

$-\overset{\overset{R_5}{|}}{C}H-CONR_7R_8$, worin $R_5$ die genannten Bedeutungen hat und $R_7$ und $R_8$ gleich oder verschieden sein können und jeweils H, $C_1$—$C_7$-Alkyl, $C_3$—$C_{12}$-Cycloalkyl, Phenyl oder Benzyl bedeuten, oder $R_7$ und $R_8$ kombiniert sind, so daß —$NR_7R_8$ den Rest eines gesättigten monocyclischen sekundären Amins darstellt;

$R_1$ $C_1$—$C_7$-Alkyl und Ar ein Phenylenrest ist, der gegebenenfalls substituiert ist durch $C_1$—$C_7$-Alkyl, $C_1$—$C_7$-Alkyl—O—$C_1$—$C_7$-alkylen-, $C_2$—$C_8$-Alkenyl, $C_1$—$C_7$-Alkyl—S—, $C_2$—$C_8$-Alkenyl—O—, $C_3$—$C_{12}$-Cycloalkyl, $C_1$—$C_7$-Alkyl—CONH—, $C_1$—$C_7$-Alkyl—CO— oder $H_2NCO$—$C_1$—$C_7$-Alkylen-, oder Ar ein Rest der Formel

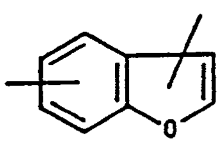,
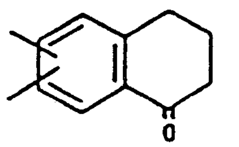,
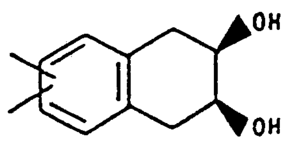,
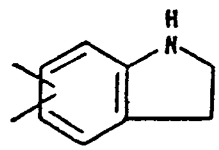,

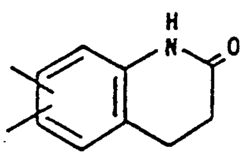,
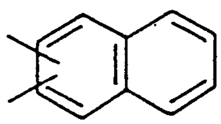 oder
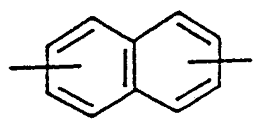.

oder Ar ein Phenylen-$C_1$—$C_3$-alkylen-phenyl-Rest ist.

2. Verbindung nach Anspruch 1, worin R $C_6$—$C_{18}$-Polycycloalkyl-$C_pH_{2p}$- ist, bestehend aus einem 0, 1 oder 2 Methylsubstituenten tragenden gesättigten alicyclischen Kohlenwasserstoffbrückensystem aus 2 oder 3 Ringen mit einer Gesamtzahl an C-Atomen im Polycycloalkylanteil von 6 bis 18, wobei p 0, 1 oder 2 bedeutet.

3. Verbindung nach Anspruch 1, worin R $C_6$—$C_{18}$-Polycycloalkenyl-$C_pH_{2p}$-, bestehend aus einem 0, 1 oder 2 Methylsubstituenten tragenden ungesättigten alicyclischen Kohlenwasserstoffbrükkensystem aus 2 oder 3 Ringen mit einer Gesamtzahl an C-Atomen im Polycycloalkenylanteil von 6 bis 18, wobei p 0, 1 oder 2 bedeutet.

4. Verbindung nach Anspruch 1, worin R Adamantyl, Bornyl, Norbornyl, Adamantylmethyl, Adamantylethyl, Norbornylmethyl, Norbornylethyl oder Norbornenyl bedeutet.

5. Verbindung nach Anspruch 1, worin R (Adamant-1-yl)ethyl, (Adamant-1-yl)methyl oder (Norborn-2-yl)methyl bedeutet.

6. Verbindung nach Anspruch 1, worin R

$$-\overset{\overset{\large R_5}{|}}{C}H-OCOR_6 \quad \text{oder} \quad -\overset{\overset{\large R_5}{|}}{C}H-CONR_7R_8$$

bedeutet, worin $R_5$, $R_6$, $R_7$ und $R_8$ die in Anspruch 1 genannten Bedeutungen haben.

7. Verbindung nach Anspruch 6, worin $R_5$ Wasserstoff und $R_6$ $C_1$—$C_7$-Alkyl ist.

8. Verbindung nach Anspruch 6, worin R Pivalyloxymethyl oder Carbamoylmethyl ist.

9. Verbindung nach einem der Ansprüche 1 bis 8, worin Ar eine Phenylengruppierung darstellt.

10. Verbindung nach Anspruch 9, worin die Phenylengruppierung unsubstituiertes Phenylen ist.

11. Verbindung nach Anspruch 10, worin die unsubstituierte Phenylengruppierung 1,4-Phenylen oder 1,3-Phenylen ist.

12. Verbindung nach einem der Ansprüche 1 bis 8, worin Ar durch $C_1$—$C_7$-Alkyl, $C_1$—$C_7$-Alkyl-O—$C_1$—$C_7$-Alkylen-, $C_2$—$C_8$-Alkenyl, $C_1$—$C_7$-Alkyl—S—, $C_2$—$C_8$-Alkenyl—O—, $C_3$—$C_{12}$-Cycloalkyl, $C_1$—$C_7$-Alkyl—CONH—, $C_1$—$C_7$-Alkyl-CO oder $H_2NCO$—$C_1$—$C_7$-Alkylen substituiertes Phenyl bedeutet.

13. Verbindung nach Anspruch 12, worin Ar Phenylen ist, das ein $CH_3CH_2$—, $CH_3$—, $CH_3OCH_2CH_2$—, $CH_2=CH$—$CH_2$—, $CH_3$—S—, $CH_2CH=CH_2$—O—,

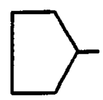 ,

$CH_3CH_2CH_2CONH$—, $CH_3CONH$—, $CH_3CO$— oder $H_2NCO$—$CH_2$— trägt.

14. Verbindung nach einem der Ansprüche 1 bis 8, worin Ar ein Rest der Formel

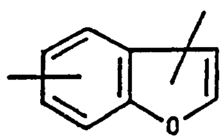 , 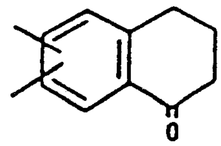 , 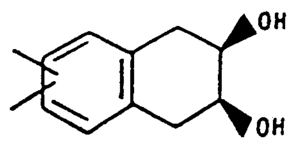 , 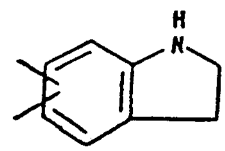 ,

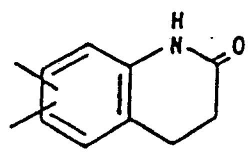 , 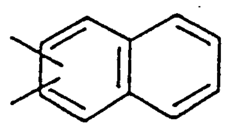 oder 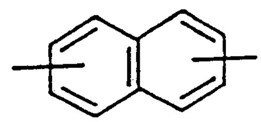 .

ist.

15. Verbindung nach Anspruch 14, worin Ar ein Rest der Formel

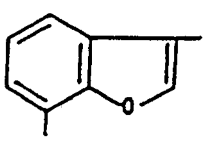 , 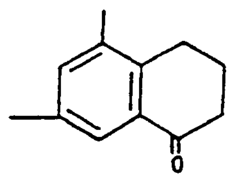 , 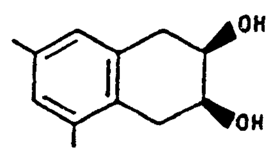 ,

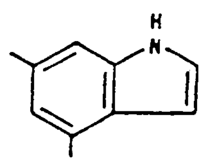 , 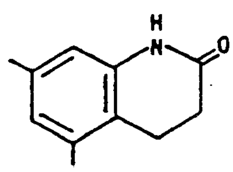 oder 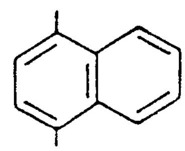 .

ist.

16. Verbindung nach einem der Ansprüche 1 bis 8, worin Ar ein Phenylen-$C_1$—$C_3$-alkylen-phenylrest ist.

17. Verbindung nach Anspruch 16, worin Ar ein Rest der Formel

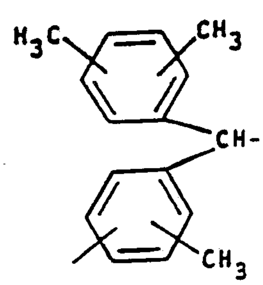

ist.

18. Verbindung nach Anspruch 1, worin die Gruppe

$$RO\overset{\overset{O}{\|}}{C}-C_nH_{2n}-$$

ein Wasserstoffatom am entsprechenden Ring eines bekannten β-Blockers ersetzt, wobei die Struktur der Verbindung der Formel I im übrigen identisch ist mit der Struktur eines bekannten β-Blockers.

19. Verbindung nach Anspruch 18, worin der bekannte β-Blocker Metoprolol, Bufurolol, Alprenolol, Bunolol, Xipranolol, Nadolol, Tiprenolol, Oxprenolol, Penbutolol, Pindolol, Carteolol, Propranolol, Acebutolol, Atenolol oder Practolol ist.

20. Verbindung nach Anspruch 1, worin die Gruppe

$$RO\overset{\overset{O}{\|}}{C}-C_nH_{2n}-$$

einen nichtkritischen Substituenten am entsprechenden Ring eines bekannten β-Blockers ersetzt, wobei die Struktur der Verbindung der Formel (I) im übrigen identisch ist mit der Struktur eines bekannten β-Blockers.

21. Verbindung nach Anspruch 20, worin der nichtkritische Substituent ein Niederalkyl- oder Niederalkyl-O-niederalkylen-Substituent ist.

22. Verbindung nach Anspruch 21, worin der nichtkritische Substituent ein $CH_3$—, $CH_3CH_2$— oder $CH_3OCH_2CH_2$-Substituent ist.

23. Verbindung nach Anspruch 20, worin der bekannte β-Blocker Metoprolol, Bufurolol oder Xipranolol ist.

24. Verbindung nach Anspruch 20, worin der nichtkritische Substituent ein Niederalkyl—CONH— oder $H_2NCO$—Niederalkylen-Substituent ist.

25. Verbindung nach Anspruch 24, worin der nichtkritische Substituent ein $CH_3CH_2CH_2CONH$—, $CH_3CONH$— oder

$$H_2N\overset{\overset{O}{\|}}{C}CH_2\text{-Substituent ist.}$$

26. Verbindung nach Anspruch 20, worin der bekannte β-Blocker Acetbutolol, Atenolol oder Practolol ist.

27. Verbindung nach einem der Ansprüche 1 bis 17, worin $R_1$ Isopropyl oder tert-Butyl ist.

28. Verbindung nach Anspruch 1, die (Adamant-1-yl)ethyl-4-(2-hydroxy-3-isopropylamino)-propoxyphenylacetat oder ein pharmazeutisch verträgliches Säureadditionssalz davon darstellt.

29. Verbindung nach Anspruch 1, die (Adamant-1-yl)methyl-4-(2-hydroxy-3-isopropylamino)-propoxyphenylacetat oder ein pharmazeutisch verträgliches Säureadditionssalz davon darstellt.

30. Verbindung nach Anspruch 1, die (Norborn-2-yl)methyl-4-(2-hydroxy-3-isopropylamino)-propoxyphenylacetat oder ein pharmazeutisch verträgliches Säureadditionssalz davon darstellt.

31. Verbindung nach Anspruch 1, die Pivalyloxymethyl-4-(2-hydroxy-3-isopropylamino)propoxyphenylacetat oder ein pharmazeutisch verträgliches Säureadditionssalz davon darstellt.

32. Verbindung nach Anspruch 1, die Carbamoylmethyl-4-(2-hydroxy-3-isopropylamino)propoxyphenylacetat oder ein pharmazeutisch verträgliches Säureadditionssalz davon darstellt.

33. Oxalatsalz einer Verbindung nach einem der Ansprüche 1 bis 32.

34. Pharmazeutische Stoffzusammensetzung, die eine Verbindung der Formel (I), wie in den vorangegangenen Ansprüchen definiert, oder ein pharmazeutisch verträgliches Säureadditionssalz davon und einen nichttoxischen pharmazeutisch verträglichen Träger enthält.

35. Pharmazeutische Stoffzusammensetzung in einer Dosiseinheitsform zur Auslösung einer β-adrenergischen Blockreaktion in einem Warmblüter, wobei diese Zusammensetzung pro Dosiseinheit eine wirksame Einheit einer β-adrenergischen blockierenden Menge einer Verbindung der Formel (I), wie in den vorangegangenen Ansprüchen definiert, oder eines pharmazeutisch verträglichen Säureadditionssalzes davon und einen nichttoxischen pharmazeutisch verträglichen Träger enthält.

36. Ophthalmische Stoffzusammensetzung, die eine Verbindung der Formel (I), wie in den vorangegangenen Ansprüchen definiert, oder ein pharmazeutisch verträgliches Säureadditionssalz davon und einen nichttoxischen ophthalmisch verträglichen Träger enthält.

37. Ophthalmische Stoffzusammensetzung in einer Dosiseinheitsform für die Behandlung von Glaucom bei einem Warmblüter, wobei die Zusammensetzung pro Dosiseinheit eine wirksame Einheit einer den Innenaugendruck vermindernden Menge einer Verbindung der Formel (I), wie in den vorangegangenen Ansprüchen definiert, oder eines pharmazeutisch verträglichen Säureadditionssalzes davon und einen nichttoxischen ophthalmisch verträglichen Träger enthält.

**Patentansprüche für den Vertragsstaaten: AT**

1. Verfahren zur Herstellung einer Verbindung der Formel

$$RO\overset{O}{\overset{\|}{C}}-C_nH_{2n}-Ar-OCH_2\overset{OH}{\overset{|}{C}}HCH_2NHR_1 \qquad (I)$$

oder eines pharmazeutisch verträglichen Säureadditionssalzes davon, worin

n 1 ist;

R $C_6$—$C_{18}$-Polycycloalkyl—$C_pH_{2p}$— ist, worin p 0, 1, 2 oder 3 ist, $C_6$—$C_{18}$-Polycycloalkenyl—$C_pH_{2p}$—, worin p die genannten Bedeutungen hat; —$CH_2$—X—$R_2$, worin X S, SO oder $SO_2$ ist und $R_2$ $C_1$—$C_7$-Alkyl oder $C_3$—$C_{12}$-Cycloalkyl ist;

$$-CH_2-\overset{+}{\underset{\underset{CH_3}{|}}{S}}-R_2,$$

worin $R_2$ die oben genannten Bedeutungen hat;

$$-\overset{\overset{R_3}{|}}{C}H-X-R_4,$$

worin X die oben genannten Bedeutungen hat, und worin $R_3$ $C_1$—$C_7$-Alkyl und $R_4$ $C_1$—$C_7$-Alkyl bedeutet, oder worin $R_3$ und $R_4$ zusammengenommen —$(CH_2)_m$— bedeuten, worin m 3 oder 4 ist und —$(CH_2)_m$— gegebenenfalls durch 1 bis 3 $C_1$—$C_7$-Alkyle substituiert ist;

$$-\overset{\overset{R_5}{|}}{C}H-OCOR_6,$$

worin $R_5$ H oder $C_1$—$C_7$-Alkyl ist und $R_6$ gegebenenfalls substituiertes $C_1$—$C_7$-Alkyl, $C_3$—$C_{12}$-Cycloalkyl, $C_3$—$C_{12}$-Cycloalkenyl oder $C_2$—$C_8$-Alkenyl ist, wobei die Substituenten ausgewählt sind aus der Gruppe, bestehend aus Halogen, $C_1$—$C_7$-Alkoxy, $C_1$—$C_7$-Alkylthio, $C_1$—$C_7$-Alkylsulfinyl, $C_1$—$C_7$-Alkylsulfonyl,

$$-NH\overset{O}{\overset{\|}{C}}-(C_1-C_7\text{-Alkyl}) \quad \text{und} \quad -O\overset{O}{\overset{\|}{C}}-(C_1-C_7\text{-Alkyl}),$$

oder $R_6$ gegebenenfalls substituiertes Phenyl oder Benzyl ist, wobei die Substituenten ausgewählt sind aus der Gruppe, bestehend aus $C_1$—$C_7$-Alkyl, $C_1$—$C_7$-Alkoxy, Halogen, Carbamoyl, $C_2$—$C_8$-Alkoxycarbonyl, $C_2$—$C_8$-Alkanoyloxy, $C_1$—$C_7$-Haloalkyl, Mono($C_1$—$C_7$-alkyl)amino, Di($C_1$—$C_7$-alkyl)amino, Mono($C_1$—$C_7$-alkyl)carbamoyl, Di($C_1$—$C_7$-alkyl)carbamoyl, $C_1$—$C_7$-Alkylthio, $C_1$—$C_7$-Alkylsulfinyl und $C_1$—$C_7$-Alkylsulfonyl;

$$-\overset{\overset{R_5}{|}}{C}H-COOR_6,$$

worin $R_5$ und $R_6$ die genannten Bedeutungen haben, oder

$$\underset{|}{R_5-CH}-CONR_7R_8,$$

worin $R_5$ die genannten Bedeutungen hat und $R_7$ und $R_8$ gleich oder verschieden sein können und jeweils H, $C_1$—$C_7$-Alkyl, $C_3$—$C_{12}$-Cycloalkyl, Phenyl oder Benzyl bedeuten, oder $R_7$ und $R_8$ kombiniert sind, so daß —$NR_7R_8$ den Rest eines gesättigten monocyclischen sekundären Amins darstellt;

$R_1$ $C_1$—$C_7$-Alkyl und Ar ein Phenylenrest ist, der gegebenenfalls substituiert ist durch $C_1$—$C_7$-Alkyl, $C_1$—$C_7$-Alkyl—O—$C_1$—$C_7$-alkylen-, $C_2$—$C_8$-Alkenyl, $C_1$—$C_7$-Alkyl—S—, $C_2$—$C_8$-Alkenyl—O—, $C_3$—$C_{12}$-Cycloalkyl, $C_1$—$C_7$-Alkyl—CONH—, $C_1$—$C_7$-Alkyl—CO— oder $H_2NCO$—$C_1$—$C_7$-Alkylen-, oder Ar ein Rest der Formel

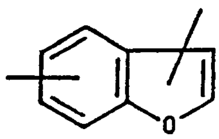, 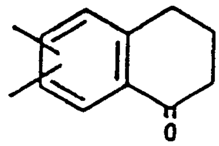, 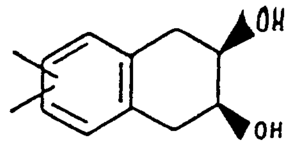, 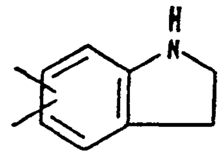,

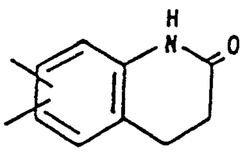, 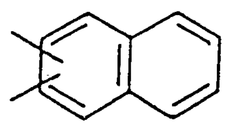 oder 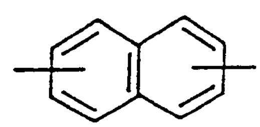.

oder Ar ein Phenylen-$C_1$—$C_3$-alkylen-phenyl-Rest ist, wobei dieses Verfahren folgende Stufen umfaßt:

a) Umsetzung der entsprechenden Verbindung der Formel

$$\overset{\displaystyle O}{\overset{\|}{ROC}}—C_nH_{2n}—Ar—OCH_2—Z \qquad (II)$$

worin R, n und Ar die obigen Bedeutungen haben und Z

$$—\overset{\displaystyle O}{\overbrace{CH—CH_2}} \quad \text{oder} \quad —\overset{\displaystyle OH}{\overset{|}{C}}HCH_2—Hal$$

ist, worin Hal ein Halogenatom ist, mit einem primären Amin der Formel

$$NH_2R_1,$$

worin $R_1$ die obige Bedeutung hat, oder

b) Veresterung der entsprechenden Säure der Formel

$$HOOC—C_nH_{2n}—Ar—OCH_2\overset{\displaystyle OH}{\overset{|}{C}}HCH_2NHR_1 \qquad (X)$$

worin n, Ar und $R_1$ die obigen Bedeutungen haben, oder des entsprechenden Säurechlorids oder -anhydrids, durch Reaktion mit einem Alkohol der Formel

$$ROH \qquad (VII),$$

worin R die obige Bedeutung hat, oder

c) für den Fall, daß eine Verbindung der Formel (I) mit einer Sulfinyl- oder Sulfonylgruppe erwünscht ist, Oxydation der entsprechenden schwefelhaltigen Verbindung der Formel (I), oder

d) für den Fall, daß ein pharmazeutisch verträgliches Säureadditionssalz erwünscht ist, Umsetzung der entsprechenden Verbindung der Formel (I) mit einer pharmazeutisch verträglichen anorganischen oder organischen Säure.

2. Verfahren nach Anspruch 1, worin R $C_6$—$C_{18}$-Polycycloalkyl-$C_pH_{2p}$- ist, bestehend aus einem 0, 1 oder 2 Methylsubstituenten tragenden gesättigten alicyclischen Kohlenwasserstoffbrückensystem aus 2 oder 3 Ringen mit einer Gesamtzahl von C-Atomen im Polycycloalkylanteil von 6 bis 18, wobei p 0, 1 oder 2 bedeutet.

3. Verfahren nach Anspruch 1, worin R $C_6$—$C_{18}$-Polycycloalkenyl-$C_pH_{2p}$-, bestehend aus einem 0, 1 oder 2 Methylsubstituenten tragenden ungesättigten alicyclischen Kohlenwasserstoffbrükkensystem aus 2 oder 3 Ringen mit einer Gesamtzahl an C-Atomen im Polycycloalkenylanteil von 6 bis 18, wobei p 0, 1 oder 2 bedeutet.

4. Verfahren nach Anspruch 1, worin R Adamantyl, Bornyl, Norbornyl, Adamantylmethyl, Adamantylethyl, Norbornylmethyl, Norbornylethyl oder Norbornenyl bedeutet.

5. Verfahren nach Anspruch 1, worin R (Adamant-1-yl)ethyl, (Adamant-1-yl)methyl oder (Norborn-2-yl)methyl bedeutet.

6. Verfahren nach Anspruch 1, worin R

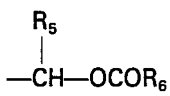 oder 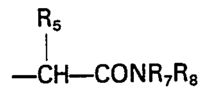

bedeutet, worin $R_5$, $R_6$, $R_7$ und $R_8$ die in Anspruch 1 genannten Bedeutungen haben.

7. Verfahren nach Anspruch 6, worin $R_5$ Wasserstoff und $R_6$ $C_1$—$C_7$-Alkyl ist.

8. Verfahren nach Anspruch 6, worin R Pivalyloxymethyl oder Carbamoylmethyl ist.

9. Verfahren nach einem der Ansprüche 1 bis 8, worin Ar eine Phenylengruppierung darstellt.

10. Verfahren nach Anspruch 9, worin die Phenylengruppierung unsubstituiertes Phenylen ist.

11. Verfahren nach Anspruch 10, worin die unsubstituierte Phenylengruppierung 1,4-Phenylen oder 1,3-Phenylen ist.

12. Verfahren nach einem der Ansprüche 1 bis 8, worin Ar durch $C_1$—$C_7$-Alkyl, $C_1$—$C_7$-Alkyl-O—$C_1$—$C_7$-Alkylen-, $C_2$—$C_8$-Alkenyl, $C_1$—$C_7$-Alkyl—S—, $C_2$—$C_8$-Alkenyl—O—, $C_3$—$C_{12}$-Cycloalkyl, $C_1$—$C_7$-Alkyl-CONH—, $C_1$—$C_7$-Alkyl-CO oder $H_2NCO$—$C_1$—$C_7$-Alkylen substituiertes Phenyl bedeutet.

13. Verfahren nach Anspruch 12, worin Ar Phenylen ist, das ein $CH_3CH_2$—, $CH_3$—, $CH_3OCH_2CH_2$—, $CH_2=CH—CH_2$—, $CH_3$—S—, $CH_2CH=CH_2$—O—,

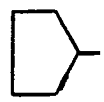 ,

$CH_3CH_2CH_2CONH$—, $CH_3CONH$—, $CH_3CO$— oder $H_2NCO$—$CH_2$— trägt.

14. Verfahren nach einem der Ansprüche 1 bis 8, worin Ar ein Rest der Formel

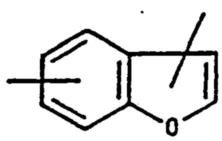 , 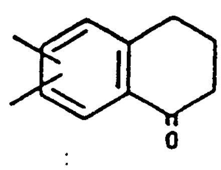 , 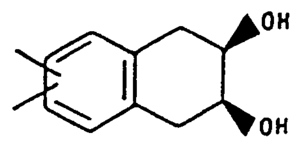 , 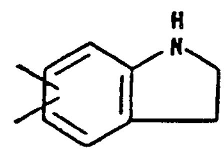 ,

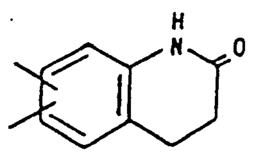 , 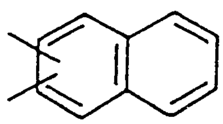 oder 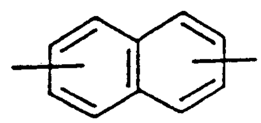 .

ist.

15. Verfahren nach Anspruch 14, worin Ar ein Rest der Formel

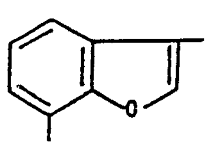 , 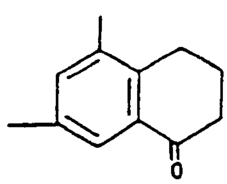 , 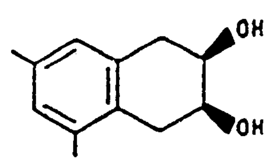 ,

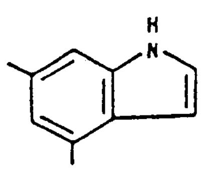 , 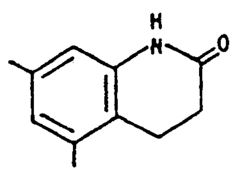 oder 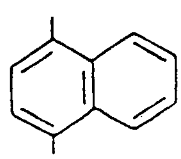 .

ist.

16. Verfahren nach einem der Ansprüche 1 bis 8, worin Ar ein Phenylen-$C_1$—$C_3$-alkylen-phenyl-Rest ist.

17. Verfahren nach Anspruch 16, worin Ar ein Rest der Formel

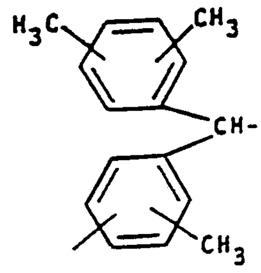

ist.

18. Verfahren nach Anspruch 1, worin die

O
‖
ROC—$C_nH_{2n}$-Gruppierung

ein Wasserstoffatom am entsprechenden Ring eines bekannten β-Blockers ersetzt, wobei die Struktur der Verbindung der Formel I im übrigen identisch ist mit der Struktur eines bekannten β-Blockers.

19. Verfahren nach Anspruch 18, worin der bekannte β-Blocker Metoprolol, Bufurolol, Alprenolol, Bunolol, Xipranolol, Nadolol, Tiprenolol, Oxprenolol, Penbutolol, Pindolol, Carteolol, Propranolol, Acebutolol, Atenolol oder Practolol ist.

20. Verfahren nach Anspruch 1, worin die

O
‖
ROC—$C_nH_{2n}$-Gruppierung

einen nichtkritischen Substituenten am entsprechenden Ring eines bekannten β-Blockers ersetzt, wobei die Struktur der Verbindung der Formel (I) im übrigen identisch ist mit der Struktur eines bekannten β-Blockers.

21. Verfahren nach Anspruch 20, worin der nichtkritische Substituent ein Niederalkyl- oder Niederalkyl-O-niederalkylen-Substituent ist.

22. Verfahren nach Anspruch 21, worin der nichtkritische Substituent ein $CH_3$—, $CH_3CH_2$— oder $CH_3OCH_2CH_2$-Substituent ist.

23. Verfahren nach Anspruch 20, worin der bekannte β-Blocker Metoprolol, Bufurolol oder Xipranolol ist.

24. Verfahren nach Anspruch 20, worin der nichtkritische Substituent ein Niederalkyl—CONH— oder $H_2NCO$—Niederalkylen-Substituent ist.

25. Verfahren nach Anspruch 24, worin der nichtkritische Substituent ein $CH_3CH_2CH_2CONH$—, $CH_3CONH$— oder

O
‖
$H_2NCCH_2$-Substituent ist.

26. Verfahren nach Anspruch 20, worin der bekannte β-Blocker Acetbutolol, Atenolol oder Practolol ist.

27. Verfahren nach einem der Ansprüche 1 bis 17, worin $R_1$ Isopropyl oder tert-Butyl bedeutet.

28. Verfahren nach Anspruch 1, worin die Verbindung der Formel (I) (Adamant-1-yl)ethyl-4-(2-hydroxy-3-isopropylamino)-propoxyphenylacetat oder ein pharmazeutisch verträgliches Säureadditionssalz davon ist.

29. Verfahren nach Anspruch 1, worin die Verbindung der Formel (I) (Adamant-1-yl)methyl-4-(2-hydroxy-3-isopropylamino)-propoxyphenylacetat oder ein pharmazeutisch verträgliches Säureadditionssalz davon ist.

30. Verfahren nach Anspruch 1, worin die Verbindung der Formel (I) (Norborn-2-yl)methyl-4-(2-hydroxy-3-isopropylamino)-propoxyphenylacetat oder ein pharmazeutisch verträglicheches Säureadditionssalz davon ist.

31. Verfahren nach Anspruch 1, worin die Verbindung der Formel (I) Pivalyloxymethyl-4-(2-hydroxy-3-isopropylamino)propoxyphenylacetat oder ein pharmazeutisch verträgliches Säureadditionssalz davon ist.

32. Verfahren nach Anspruch 1, worin die Verbindung der Formel (I) Carbamoylmethyl-4-(2-hydroxy-3-isopropylamino)propoxyphenylacetat oder ein pharmazeutisch verträgliches Säureadditionssalz davon ist.

33. Verfahren nach einem der Ansprüche 1 bis 32, worin die Verbindung der Formel (I) mit Oxalsäure zum entsprechenden Oxalatsalz umgesetzt wird.

**Revendications pour les Etats contractants: BE CH DE FR GB LU NL SE**

1. Composé de formule

$$\begin{array}{c} \quad O \qquad\qquad\qquad\quad OH \\ \quad \| \qquad\qquad\qquad\quad | \\ ROC—C_nH_{2n}—Ar—OCH_2CHCH_2NHR_1 \end{array} \qquad (I)$$

ou un sel d'addition d'acide acceptable du point de vue pharmaceutique de celui-ci, dans lequel:

n est égal à 1;

R est un polycycloalkyle en $C_6—C_{18}—C_pH_{2p}$ dans lequel p est égal à 0, 1, 2 ou 3; un polycycloalcényle en $C_6—C_{18}—C_pH_{2p}$ dans lequel p est défini comme ci-dessus, $—CH_2—X—R_2$ dans lequel X est S, SO ou $SO_2$ et $R_2$ est un alkyle en $C_1—C_7$ ou un cycloalkyle en $C_3—C_{12}$;

$$\begin{array}{c} \quad + \\ —CH_2—S—R_2 \\ \quad | \\ \quad CH_3 \end{array}$$

dans lequel $R_2$ est défini comme ci-dessus;

$$\begin{array}{c} R_3 \\ | \\ —CH—X—R_4 \end{array}$$

dans lequel X est défini comme ci-dessus et dans lequel $R_3$ est un alkyle en $C_1—C_7$ et $R_4$ est un alkyle en $C_1—C_7$ ou dans lequel $R_3$ et $R_4$ pris ensemble représentent $—(CH_2)_m—$ dans lequel m est 3 ou 4 et $—(CH_2)_m—$ est éventuellement substitué par 1 à 3 alkyles en $C_1—C_7$;

$$\begin{array}{c} R_5 \\ | \\ —CH—OCOR_6 \end{array}$$

dans lequel $R_5$ est l'hydrogène ou un alkyle en $C_1—C_7$ et $R_6$ est un alkyle en $C_1—C_7$, un cycloalkyle en $C_3—C_{12}$, un cycloalcényle en $C_3—C_{12}$ ou un alcényle en $C_2—C_8$ substitué ou non substitué, les substituants étant choisis dans le groupe formé par les halogènes, alcoxy en $C_1—C_7$, alkylthio en $C_1—C_7$, alkylsulfinyle en $C_1—C_7$, alkylsulfonyle en $C_1—C_7$,

$$\begin{array}{c} \quad O \\ \quad \| \\ —NHC\text{-(alkyle en } C_1—C_7\text{)} \end{array} \text{ et } \begin{array}{c} \quad O \\ \quad \| \\ —OC—\text{(alkyle en } C_1—C_7\text{)}, \end{array}$$

ou $R_6$ est un phényle ou benzyle substitué ou non substitué, les substituants étant choisi dans le groupe formé par alkyle en $C_1—C_7$, alcoxy en $C_1—C_7$, halogène, carbamoyle, alcoxycarbonyle en $C_2—C_8$, alcanoyloxy en $C_2—C_8$, halogénoalkyle en $C_1—C_7$, mono(alkyle en $C_1—C_7$)amino, di(alkyle en $C_1—C_7$)amino, mono(alkyle en $C_1—C_7$)carbamoyle, di(alkyle en $C_1—C_7$)carbamoyle, alkylthio en $C_1—C_7$, alkylsulfinyle en $C_1—C_7$ et alkylsulfonyle en $C_1—C_7$;

$$\begin{array}{c} R_5 \\ | \\ —CH—COOR_6 \end{array}$$

dans lequel $R_5$ et $R_6$ sont définis comme ci-dessus, ou

$$\begin{array}{c} R_5 \\ | \\ —CH—CONR_7R_8 \end{array}$$

dans lequel $R_5$ est défini comme ci-dessus et $R_7$ et $R_8$, qui sont identiques ou différents, sont chacun l'hydrogène, alkyle en $C_1—C_7$, cycloalkyle en $C_3—C_{12}$, phényle ou benzyle, ou $R_7$ et $R_8$ sont combinés de telle façon que $—NR_7R_8$ représente le résidu d'une amine secondaire monocyclique saturée,

$R_1$ est un alkyle en $C_1—C_7$; et

Ar est un radical phénylène qui n'est pas substitué ou qui est substitué par un alkyle en $C_1—C_7$, alkyle en $C_1—C_7—O—$alkylène en $C_1—C_7$, alcényle en $C_2—C_8$, alkyle en $C_1—C_7—S—$, alcényle en $C_2—C_8—O—$,

cycloalkyle en $C_3$—$C_{12}$, alkyle en $C_1$—$C_7$—CONH—, alkyle en $C_1$—$C_7$—CO— ou $H_2NCO$-alkylène en $C_1$—$C_7$; ou Ar est un radical de formule

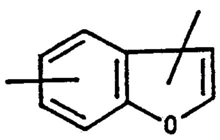, 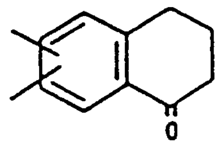, 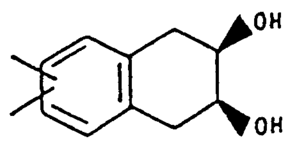, 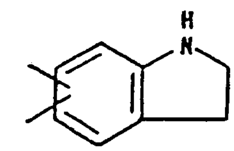,

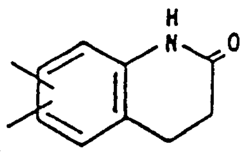, 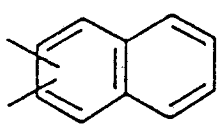 ou 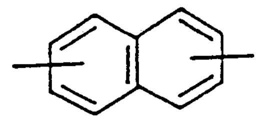.

ou Ar est un radical phénylène-alkylène en $C_1$—$C_3$-phényle.

2. Composé selon la revendication 1, dans lequel R est un polycycloalkyle en $C_6$—$C_{18}$—$C_pH_{2p}$— consistant en un système hydrocarboné alicyclique saturé ponté à deux ou trois cycles portant zéro, un ou deux substituants méthyle et ayant un total de 6 à 18 atomes de carbone dans la partie polycycloalkyle et p est égal à zéro, un ou deux.

3. Composé selon la revendication 1, dans lequel R est un polycycloalcényle en $C_6$—$C_{18}$—$C_pH_{2p}$— consistant en un système hydrocarboné alicyclique insaturé ponté à deux ou trois cycles portant zéro, un ou deux substituants méthyle ayant un total de 6 à 18 atomes de carbone dans la partie polycycloalcényle et p est égal à zéro, un ou deux.

4. Composé selon la revendication 1, dans lequel R est adamantyle, bornyle, norbornyle, adamantylméthyle, adamantyléthyle, norbornylméthyle, norbornyléthyle ou norbornényle.

5. Composé selon la revendication 1, dans lequel R est (adamant-1-yl)éthyle, (adamant-1-yl)méthyle ou (norborn-2-yl)-méthyle.

6. Composé selon la revendication 1, dans lequel R est

$$-\underset{|}{\overset{R_5}{}}\!\!CH-OCOR_6 \quad \text{ou} \quad -\overset{R_5}{\overset{|}{C}}H-CONR_7R_8,$$

où $R_5$, $R_6$, $R_7$ et $R_8$ sont tels que définis dans la revendication 1.

7. Composé selon la revendication 6, dans lequel $R_5$ est l'hydrogène et $R_6$ est un alkyle en $C_1$—$C_7$.

8. Composé selon la revendication 6, dans lequel R est un pivalyloxyméthyle ou carbamoylméthyle.

9. Composé selon l'une quelconque des revendications 1 à 8, dans lequel Ar comprend un groupe phénylène.

10. Composé selon la revendication 9, dans lequel ledit groupe phénylène est un phénylène est un phénylène non substitué.

11. Composé selon la revendication 10, dans lequel le groupe phénylène non substitué est le 1,4-phénylène ou le 1,3-phénylène.

12. Composé selon l'une quelconque des revendications 1 à 8, dans lequel Ar est un phénylène substitué par un alkyle en $C_1$—$C_7$, un alkyle en $C_1$—$C_7$—O—alkylène en $C_1$—$C_7$, alcényle en $C_2$—$C_8$, alkyle en $C_1$—$C_7$—S—, alcényle en $C_2$—$C_8$—O—, cycloalkyle en $C_3$—$C_{12}$, alkyle en $C_1$—$C_7$—CONH—, alkyle en $C_1$—$C_7$—CO— ou $H_2NCO$-alkylène en $C_1$—$C_7$.

13. Composé selon la revendication 12, dans lequel Ar est un phénylène portant un groupe $CH_3CH_2$—, $CH_3$—, $CH_3OCH_2CH_2$—, $CH_2$=CH—$CH_2$—, $CH_3$—S—, $CH_2CH$=$CH_2$—O—,

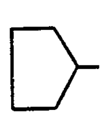,

$CH_3CH_2CH_2CONH$—, $CH_3CONH$—, $CH_3CO$— ou $H_2NCO$—$CH_2$—.

14. Composé selon l'une quelconque des revendications 1 à 8, dans lequel Ar est un radical de formule

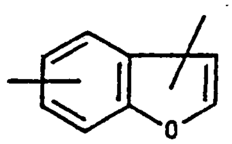, 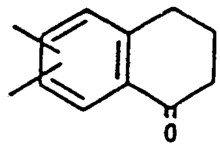, 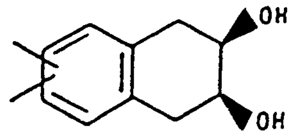, 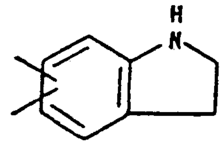,

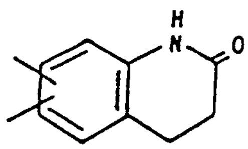, 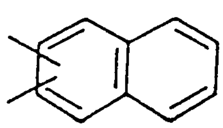 ou 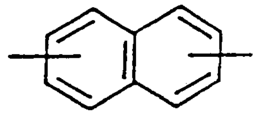.

15. Composé selon la revendication 14, dans lequel Ar est un radical de formule

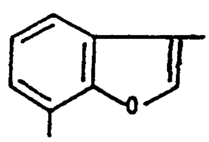, 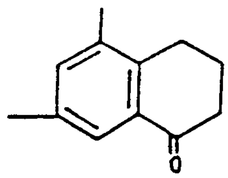, 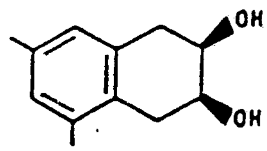,

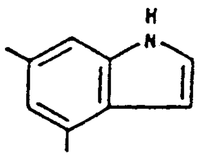, 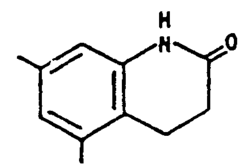 ou 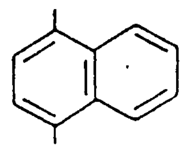.

16. Composé selon l'une quelconque des revendications 1 à 8, dans lequel Ar est un radical phénylène-alkylène en $C_1$—$C_3$-phényle.

17. Composé selon la revendication 16, dans lequel Ar est un radical de formule

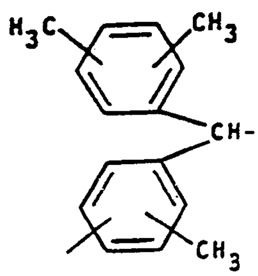

18. Composé selon la revendication 1, dans lequel le groupe

$$RO\overset{\overset{\displaystyle O}{\|}}{C}—C_nH_{2n}—$$

remplace un atome d'hydrogène sur le cycle correspondant d'un β-bloquant connu, la structure du composé de formule (I) étant par ailleurs identique à la structure d'un β-bloquant connu.

19. Composé selon la revendication 18, dans lequel le β-bloquant connu est le métoprolol, bufurolol, alprénolol, bunolol, xipranolol, nadolol, tiprénolol, oxprénolol, penbutolol, pendolol, cartéolol, propranolol, acébutolol, aténolol ou le practolol.

20. Composé selon la revendication 1, dans lequel le groupe

$$RO\overset{\overset{\displaystyle O}{\|}}{C}—C_nH_{2n}—$$

remplace un substituant non critique sur le cycle correspondant d'un β-bloquant connu, la structure du

composé de formule (I) étant par ailleurs identique à la structure d'un β-bloquant connu.

21. Composé selon la revendication 20, dans lequel le substituant non critique est un substituant alkyle inférieur ou alkyle inférieur-O-alkylène inférieur.

22. Composé selon la revendication 21, dans lequel le substituant non critique est un substituant $CH_3$—, $CH_3CH_2$— ou $CH_3OCH_2CH_2$—.

23. Composé selon la revendication 20, dans lequel le β-bloquant connu est le métoprolol, le bufurolol ou le xipranolol.

24. Composé selon la revendication 20, dans lequel le substituant non critique est un substituant alkyle inférieur—CONH— ou $H_2NCO$-alkylène inférieur.

25. Composé selon la revendication 24, dans lequel le substituant non critique est un substituant $CH_3CH_2CH_2CONH$—, $CH_3CONH$— ou

$$H_2N\overset{\overset{\displaystyle O}{\|}}{C}CH_2—.$$

26. Composé selon la revendication 20, dans lequel le β-bloquant connu et l'acébutolol, l'aténolol ou le practolol.

27. Composé selon l'une quelconque des revendications 1 à 17, dans lequel $R_1$ est l'isopropyle ou le tert-butyle.

28. Composé selon la revendication 1, qui est le 4-(2-hydroxy-3-isopropylamino)-propoxyphénylacétate de (adamant-1-yl)éthyle ou un sel d'addition d'acide acceptable du point de vue pharmaceutique de celui-ci.

29. Composé selon la revendication 1, qui est le 4-(2-hydroxy-3-isopropylamino)-propoxyphénylacétate de (adamant-1-yl)méthyle ou un sel d'addition d'acide acceptable du point de vue pharmaceutique de celui-ci.

30. Composé selon la revendication 1, qui est le 4-(2-hydroxy-3-isopropylamino)-propoxyphénylacétate de (norborn-2-yl)méthyle ou un sel d'addition d'acide acceptable du point de vue pharmaceutique de celui-ci.

31. Composé selon la revendication 1, qui est le 4-(2-hydroxy-3-isopropylamino)-propoxyphénylacétate de pivalyloxyméthyle ou un sel d'addition d'acide acceptable du point de vue pharmaceutique de celui-ci.

32. Composé selon la revendication 1, qui est le 4-(2-hydroxy-3-isopropylamino)-propoxyphénylacétate de carbamoylméthyle ou un sel d'addition d'acide acceptable du point de vue pharmaceutique de celui-ci.

33. Oxalate d'un composé tel que défini par l'une quelconque des revendications 1 à 32.

34. Composition de matière pharmaceutique comprenant un composé de formule (I) tel que défini par l'une quelconque des revendications précédentes, ou un sel d'addition d'acide acceptable du point de vue pharmaceutique de celui-ci, et un véhicule non toxique acceptable du point de vue pharmaceutique pour celui-ci.

35. Composition de matière pharmaceutique sous forme de doses unitaires utilisable pour provoquer une réponse de blocage β-adrénergique chez un animal à sang chaud, ladite composition comprenant par dose unitaire une quantité unitaire efficace de blocage β-adrénergique d'un composé de formule (I) tel que défini par l'une quelconque des revendications précédentes, ou un sel d'addition d'acide acceptable du point de vue pharmaceutique de celui-ci, et un véhicule non toxique acceptable du point de vue pharmaceutique pour celui-ci.

36. Composition de matière ophtalmique comprenant un composé de formule (I) tel que défini par l'une quelconque des revendications précédentes, ou un sel d'addition d'acide acceptable du point de vue pharmaceutique de celui-ci, et un véhicule non toxique acceptable du point de vue ophtalmique pour celui-ci.

37. Composition de matière ophtalmique sous forme de doses unitaires utilisable dans le traitement des glaucomes chez un animal à sang chaud, ladite composition comprenant par dose unitaire une quantité unitaire efficace de diminution de la pression intra-oculaire d'un composé de formule (I) tel que défini par l'une quelconque des revendications précédentes ou d'un sel d'addition d'acide acceptable du point de vue pharmaceutique de celui-ci, et un véhicule non toxique acceptable du point de vue ophtalmique pour celui-ci.

**Revendications pour l'Etat Contractant: AT**

1. Procédé de préparation d'un composé de formule

$$RO\overset{\overset{\displaystyle O}{\|}}{C}—C_nH_{2n}—Ar—OCH_2\overset{\overset{\displaystyle OH}{|}}{C}HCH_2NHR_1 \qquad (I)$$

ou d'un sel d'addition d'acide acceptable du point de vue pharmaceutique de celui-ci, dans lequel:

n est égal à 1;

R est un polycycloalkyle en $C_6$—$C_{18}$—$C_pH_{2p}$ dans lequel p est égal à 0, 1, 2 ou 3; un polycycloalcényle en $C_6$—$C_{18}$—$C_pH_{2p}$ dans lequel p est défini comme ci-dessus, —$CH_2$—X—$R_2$ dans lequel X est S, SO ou $SO_2$ et $R_2$ est un alkyle en $C_1$—$C_7$ ou un cycloalkyle en $C_3$—$C_{12}$;

$$-CH_2-\overset{+}{\underset{CH_3}{\underset{|}{S}}}-R_2$$

dans lequel $R_2$ est défini comme ci-dessus;

$$-\overset{R_3}{\overset{|}{C}}H-X-R_4$$

dans lequel X est défini comme ci-dessus et dans lequel $R_3$ est un alkyle en $C_1$—$C_7$ et $R_4$ est un alkyle en $C_1$—$C_7$ ou dans lequel $R_3$ et $R_4$ pris ensemble représentent —$(CH_2)_m$— dans lequel m est 3 ou 4 et —$(CH_2)_m$— est éventuellement substitué par 1 à 3 alkyles en $C_1$—$C_7$;

$$-\overset{R_5}{\overset{|}{C}}H-OCOR_6$$

dans lequel $R_5$ est l'hydrogène ou un alkyle en $C_1$—$C_7$ et $R_6$ est un alkyle en $C_1$—$C_7$, un cycloalkyle en $C_3$—$C_{12}$, un cycloalcényle en $C_3$—$C_{12}$ ou un alcényle en $C_2$—$C_8$ substitué ou non substitué, les substituants étant choisis dans le groupe formé par les halogènes, alcoxy en $C_1$—$C_7$, alkylthio en $C_1$—$C_7$, alkylsulfinyle en $C_1$—$C_7$, alkylsulfonyle en $C_1$—$C_7$,

$$-NH\overset{O}{\overset{\|}{C}}\text{-(alkyle en } C_1-C_7) \text{ et } -O\overset{O}{\overset{\|}{C}}-\text{(alkyle en } C_1-C_7),$$

ou $R_6$ est un phényle ou benzyle substitué ou non substitué, les substituants étant choisi dans le groupe formé par alkyle en $C_1$—$C_7$, alcoxy en $C_1$—$C_7$, halogène, carbamoyle, alcoxycarbonyle en $C_2$—$C_8$, alcanoyloxy en $C_2$—$C_8$, halogénoalkyle en $C_1$—$C_7$, mono(alkyle en $C_1$—$C_7$)amino, di(alkyle en $C_1$—$C_7$)amino, mono(alkyle en $C_1$—$C_7$)carbamoyle, di(alkyle en $C_1$—$C_7$)carbamoyle, alkylthio en $C_1$—$C_7$, alkylsulfinyle en $C_1$—$C_7$ et alkylsulfonyle en $C_1$—$C_7$;

$$-\overset{R_5}{\overset{|}{C}}H-COOR_6$$

dans lequel $R_5$ et $R_6$ sont définis comme ci-dessus, ou

$$-\overset{R_5}{\overset{|}{C}}H-CONR_7R_8$$

dans lequel $R_5$ est défini comme ci-dessus et $R_7$ et $R_8$, qui sont identiques ou différents, sont chacun l'hydrogène, alkyle en $C_1$—$C_7$, cycloalkyle en $C_3$—$C_{12}$, phényle ou benzyle, ou $R_7$ et $R_8$ sont combinés de telle façon que —$NR_7R_8$ représente le résidu d'une amine secondaire monocyclique saturée,

$R_1$ est un alkyle en $C_1$—$C_7$; et

Ar est un radical phénylène qui n'est pas substitué ou qui est substitué par un alkyle en $C_1$—$C_7$, alkyle en $C_1$—$C_7$—O—alkylène en $C_1$—$C_7$, alcényle en $C_2$—$C_8$, alkyle en $C_1$—$C_7$—S—, alcényle en $C_2$—$C_8$—O—, cycloalkyle en $C_3$—$C_{12}$, alkyle en $C_1$—$C_7$—CONH—, alkyle en $C_1$—$C_7$—CO— ou $H_2NCO$-alkylène en $C_1$—$C_7$; ou Ar est un radical de formule

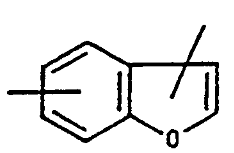 , 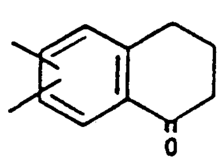 , 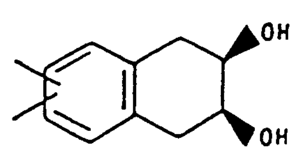 , 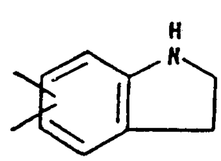 .

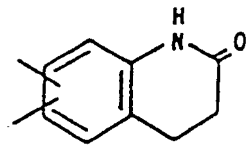 , 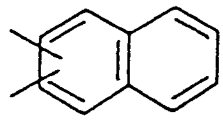 ou 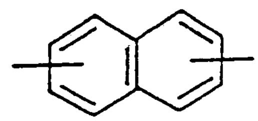 .

ou Ar est un radical phénylène-alkylène en $C_1$—$C_3$-phényle; ledit procédé comprenant:

(a) la réaction du composé correspondant de formule

$$ROC(=O)—C_nH_{2n}—Ar—OCH_2—Z \qquad (II)$$

dans laquelle R, n et Ar sont définis comme ci-dessus et Z est

$$—CH(—O—)CH_2 \quad ou \quad —CH(OH)CH_2—Hal$$

dans lesquelles Hal est un atome d'halogène, avec une amine primaire de formula

$$NH_2R_1$$

dans laquelle $R_1$ est défini comme ci-dessus; ou

(b) l'estérification de l'acide correspondant de formule

$$HOOC—C_nH_{2n}—Ar—OCH_2CH(OH)CH_2NHR_1 \qquad (X)$$

dans laquelle n, Ar et $R_1$ sont définis comme ci-dessus, ou du chlorure d'acide ou de l'anhydride d'acide correspondant, par réaction avec un alcool de formule

$$ROH \qquad (VII)$$

dans laquelle R est défini comme ci-dessus; ou

(c) lorsqu'un composé de formule (I) contenant un groupe sulfinyle ou sulfonyle est souhaité, l'oxydation du composé contenant du soufre correspondant de formule (I); ou

(d) lorsqu'un sel d'addition d'acide acceptable du point de vue pharmaceutique est souhaité, la réaction du composé correspondant de formule (I) avec un acide inorganique ou organique acceptable du point de vue pharmaceutique.

2. Procédé selon la revendication 1, dans lequel R est un polycycloalkyle en $C_6$—$C_{18}$—$C_pH_{2p}$— consistant en un système hydrocarboné alicyclique saturé ponté à deux ou trois cycles portant zéro, un ou deux substituants méthyle et ayant un total de 6 à 18 atomes de carbone dans la partie polycycloalkyle et p est égal à zéro, un ou deux.

3. Procédé selon la revendication 1, dans lequel R est un polycycloalcényle en $C_6$—$C_{18}$—$C_pH_{2p}$— consistant en un système hydrocarboné alicyclique insaturé ponté à deux ou trois cycles portant zéro, un ou deux substituants méthyle ayant un total de 6 à 18 atomes de carbone dans la partie polycycloalcényle et p est égal à zéro, un ou deux.

4. Procédé selon la revendication 1, dans lequel R est adamantyle, bornyle, norbornyle, adamantylméthyle, adamantyléthyle, norbornylméthyle, norbornyléthyle ou norbornényle.

5. Procédé selon la revendication 1, dans lequel R est (adamant-1-yl)éthyle, (adamant-1-yl)méthyle ou (norborn-2-yl)-méthyle.

6. Procédé selon la revendication 1, dans lequel R est

$$—CH(R_5)—OCOR_6 \quad ou \quad —CH(R_5)—CONR_7R_8,$$

où $R_5$, $R_6$, $R_7$ et $R_8$ sont tels que définis dans la revendication 1.

7. Procédé selon la revendication 6, dans lequel $R_5$ est l'hydrogène et $R_6$ est un alkyle en $C_1$—$C_7$.

8. Procédé selon la revendication 6, dans lequel R est un pivalyloxyméthyle ou carbamoylméthyle.

9. Procédé selon l'une quelconque des revendications 1 à 8, dans lequel Ar comprend un groupe phénylène.

10. Procédé selon la revendication 9, dans lequel ledit groupe phénylène est un phénylène non substitué.

11. Procédé selon la revendication 10, dans lequel le groupe phénylène non substitué est le 1,4-phénylène ou le 1,3-phénylène.

12. Procédé selon l'une quelconque des revendications 1 à 8, dans lequel Ar est un phénylène substitué par un alkyle en $C_1$—$C_7$, un alkyle en $C_1$—$C_7$—O—alkylène en $C_1$—$C_7$, alcényle en $C_2$—$C_8$, alkyle en $C_1$—$C_7$—S—, alcényle en $C_2$—$C_8$—O—, cycloalkyle en $C_3$—$C_{12}$, alkyle en $C_1$—$C_7$—CONH—, alkyle en $C_1$—$C_7$—CO— ou $H_2NCO$-alkylène en $C_1$—$C_7$.

13. Procédé selon la revendication 12, dans lequel Ar est un phénylène portant un groupe $CH_3CH_2$—, $CH_3$—, $CH_3OCH_2CH_2$—, $CH_2$=CH—$CH_2$—, $CH_3$—S—, $CH_2CH$=$CH_2$—O—,

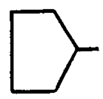 ,

$CH_3CH_2CH_2CONH$—, $CH_3CONH$—, $CH_3CO$— ou $H_2NCO$—$CH_2$—.

14. Procédé selon l'une quelconque des revendications 1 à 8, dans lequel Ar est un radical de formule

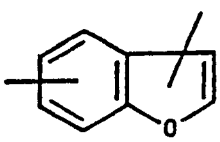 , 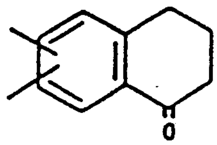 , 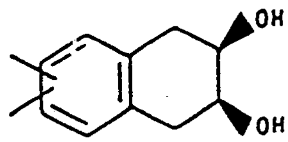 , 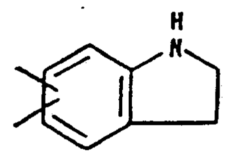 ,

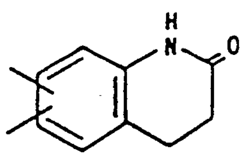 , 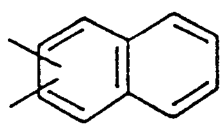 ou 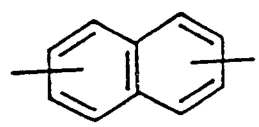 .

15. Procédé selon la revendication 14, dans lequel Ar est un radical de formule

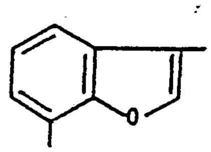 , 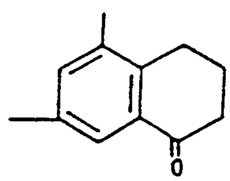 , 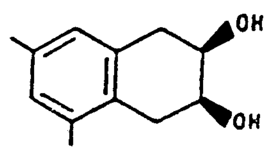 ,

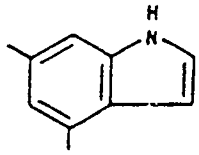 , 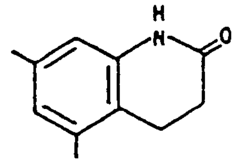 ou 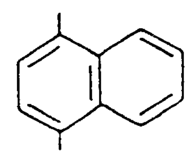 .

16. Procédé selon l'une quelconque des revendications 1 à 8, dans lequel Ar est un radical phénylène-alkylène en $C_1$—$C_3$-phényle.

17. Procédé selon la revendication 16, dans lequel Ar est un radical de formule

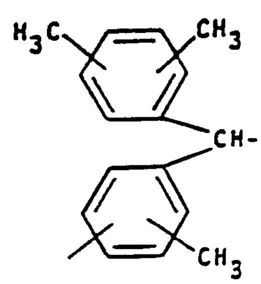

18. Procédé selon la revendication 1, dans lequel le groupe

$$RO\overset{\overset{O}{\|}}{C}—C_nH_{2n}—$$

remplace un atome d'hydrogène sur le cycle correspondant d'un β-bloquant connu, la structure du composé de formule (I) étant par ailleurs identique à la structure d'un β-bloquant connu.

19. Procédé selon la revendication 18, dans lequel le β-bloquant connu est le métoprolol, bufurolol, alprénolol, bunolol, xipranolol, nadolol, tiprénolol, oxprénolol, penbutolol, pendolol, cartéolol, propranolol, acébutolol, aténolol ou le practolol.

20. Procédé selon la revendication 1, dans lequel le groupe

$$RO\overset{\overset{O}{\|}}{C}—C_nH_{2n}—$$

remplace un substituant non critique sur le cycle correspondant d'un β-bloquant connu, la structure du composé de formule (I) étant par ailleurs identique à la structure d'un β-bloquant connu.

21. Procédé selon la revendication 20, dans lequel le substituant non critique est un substituant alkyle inférieur ou alkyle inférieur-O-alkylène inférieur.

22. Procédé selon la revendication 21, dans lequel le substituant non critique est un substituant $CH_3—$, $CH_3CH_2—$ ou $CH_3OCH_2CH_2—$.

23. Procédé selon la revendication 20, dans lequel le β-bloquant connu est le métoprolol, le bufurolol ou le xipranolol.

24. Procédé selon la revendication 20, dans lequel le substituant non critique est un substituant alkyle inférieur—CONH— ou $H_2NCO$-alkylène inférieur.

25. Procédé selon la revendication 24, dans lequel le substituant non critique est un substituant $CH_3CH_2CH_2CONH—$, $CH_3CONH—$ ou

$$H_2N\overset{\overset{O}{\|}}{C}CH_2—.$$

26. Procédé selon la revendication 20, dans lequel le β-bloquant connu est l'acébutolol, l'aténolol ou le practolol.

27. Procédé selon l'une quelconque des revendications 1 à 17, dans lequel $R_1$ est l'isopropyle ou tert-butyle.

28. Procédé selon la revendication 1, dans lequel le composé de formule (I) est le 4-(2-hydroxy-3-isopropylamino)-propoxyphénylacétate de (adamant-1-yl)éthyle ou un sel d'addition d'acide acceptable du point de vue pharmaceutique de celui-ci.

29. Procédé selon la revendication 1, dans lequel le composé de formule (I) est le 4-(2-hydroxy-3-isopropylamino)-propoxyphénylacétate de (adamant-1-yl)méthyle ou un sel d'addition d'acide acceptable du point de vue pharmaceutique de celui-ci.

30. Procédé selon la revendication 1, dans lequel le composé de formule (I) est le 4-(2-hydroxy-3-isopropylamino)-propoxyphénylacétate de (norborn-2-yl)méthyle ou un sel d'addition d'acide acceptable du point de vue pharmaceutique de celui-ci.

31. Procédé selon la revendication 1, dans lequel le composé de formule (I) est le 4-(2-hydroxy-3-isopropylamino)-propoxyphénylacétate de pivalyloxyméthyle ou un sel d'addition d'acide acceptable du point de vue pharmaceutique de celui-ci.

32. Procédé selon la revendication 1, dans lequel le composé de formule (I) est le 4-(2-hydroxy-3-isopropylamino)-propoxyphénylacétate de carbamoylméthyle ou un sel d'addition d'acide acceptable du point de vue pharmaceutique de celui-ci.

33. Procédé selon l'une quelconque des revendications 1 à 32, dans lequel le composé de formule (I) est mis à réagir avec l'acide oxalique pour obtenir l'oxalate correspondant.